# EUROPEAN PATENT SPECIFICATION

(11) **EP 2 163 888 B1**
(45) Date of publication and mention of the grant of the patent: **26.12.2018**
(21) Application number: 08777223.2
(22) Date of filing: 13.06.2008
(51) Int. Cl.: G01N 27/327, C12Q 1/00

(54) **ENZYME ELECTRODE AND ENZYME SENSOR**
ENZYMELEKTRODE UND ENZYMSENSOR
ELECTRODE ENZYMATIQUE ET CAPTEUR ENZYMATIQUE

(30) Priority: 15.06.2007 JP 2007159311; 28.09.2007 JP 2007255402
(43) Date of publication of application: 17.03.2010
(73) Proprietor: Funai Electric Co., Ltd., Daito-shi Osaka 574-0013 (JP); National Institute of Advanced Industrial Science and Technology, Tokyo 100-8921 (JP)
(72) Inventor: SHIMOMURA, Takeshi, Tsukuba-shi, Ibaraki 305-0047 (JP); SUMIYA, Touru, Tsukuba-shi, Ibaraki 305-0047 (JP); MASUDA, Yuichiro, Tsukuba-shi, Ibaraki 305-0047 (JP); ONO, Masatoshi, Tsukuba-shi, Ibaraki 305-0047 (JP); ITOH, Tetsuji, Sendai-shi, Miyagi 983-8551 (JP); MIZUKAMI, Fujio, Sendai-shi, Miyagi 983-8551 (JP)
(74) Representative: Grünecker Patent- und Rechtsanwälte PartG mbB
(86) International application number: PCT/JP2008/060912
(87) International publication number: WO 2008/153157

(56) References cited:
- WO-A1-2006/132294
- JP-A- 2005 061 961
- DAI ET AL: "Direct electrochemistry of glucose oxidase immobilized on a hexagonal mesoporous silica-MCM-41 matrix", BIOELECTROCHEMISTRY, ELESEVIER, AMSTERDAM, NL, vol. 70, no. 2, 3 April 2007 (2007-04-03), pages 250-256, XP022015479, ISSN: 1567-5394, DOI: 10.1016/J.BIOELECHEM.2006.09.009
- BAI ET AL: "Gold nanoparticles-mesoporous silica composite used as an enzyme immobilization matrix for amperometric glucose biosensor construction", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, vol. 124, no. 1, 16 May 2007 (2007-05-16), pages 179-186, XP022080520, ISSN: 0925-4005, DOI: 10.1016/J.SNB.2006.12.020
- ZHIHUI DAI ET AL: "Detection of Trace Phenol Based on Mesoporous Silica Derived Tyrosinase-Peroxidase Biosensor", ELECTROANALYSIS, vol. 17, no. 17, 1 September 2005 (2005-09-01), pages 1571-1577, XP055127290, ISSN: 1040-0397, DOI: 10.1002/elan.200403256
- TETSUJI ITOH ET AL: "Effective immobilization of subunit protein in mesoporous silica modified with ethanol", BIOTECHNOLOGY AND BIOENGINEERING, vol. 97, no. 1, 1 January 2007 (2007-01-01), pages 200-205, XP055127550, ISSN: 0006-3592, DOI: 10.1002/bit.21231
- MARTIN HARTMANN: "Ordered Mesoporous Materials for Bioadsorption and Biocatalysis", CHEMISTRY OF MATERIALS, vol. 17, no. 18, 1 September 2005 (2005-09-01), pages 4577-4593, XP055127248, ISSN: 0897-4756, DOI: 10.1021/cm0485658
- TAKAHASHI H. ET AL.: 'Koso no Bunshi Shinka to Mesoporous Kukan eno Koteika ni yoru Cho Anteika' ZEOLITE NEWS LETTER vol. 23, no. 1, 10 March 2006, pages 18 - 26, XP008169397

## Description

### Field of Invention

The present invention relates to an enzyme electrode and an enzyme sensor using the enzyme electrode.

### Background of the Art

An enzyme sensor is known as a method for quantifying the existing amount of a specific component (target substance) in a multicomponent sample such as in an environment or a biological sample at high accuracy by utilizing excellent substrate specificity of enzymes. For example, in the field of clinical science, an enzyme electrode for the enzyme sensor which enables selective detection of glucose, urea, uric acid, or the like is researched and developed. Since enzymes have the high substrate specificity, the enzymes can selectively react with the target substance (substrate) in the sample without giving a complicated pre-treatment to the sample to be measured.

The enzyme electrode includes an electrode and an enzyme immobilized film in general. An enzyme sensor using the enzyme electrode is capable of measuring the concentration of the substrate on which the enzymes act specifically, for example, by electrochemically detecting change of the substance, which results from the reaction of the target substance in the sample with the enzymes, as the amount of change of an electric signal by using the electrode.

For example, the enzyme sensor (glucose sensor) using the enzyme electrode made by attaching the enzyme immobilized film, in which glucose oxidase is immobilized, onto a diaphragm electrode is capable of measuring the concentration of a bio-substrate by monitoring an active substance of an electrode such as a hydrogen peroxide solution produced by an enzyme reaction; a substrate + O₂ - (an enzyme) → a product + H₂O₂, and oxygen consumed thereby.

Enzymes have been immobilized in various kinds of carriers for an enzyme sensor using an enzyme electrode in order to improve detection sensitivity and detection efficiency thereof. Conventionally, as methods for immobilizing enzymes on an enzyme electrode, a cross-linking method in which enzymes react with a reagent which has two or more functional groups, a carrier binding method in which enzymes (proteins) bind an insoluble carrier, an entrapment method such as an immobilization-by-entrapment method in which enzymes are entrapped into a fine lattice of gel and a microcapsule method in which enzymes are coated with a semi-transparent polymer film, a physical adsorption method, and the like are known.

Among the above-mentioned methods, the cross-linking method is widely employed owing to its simple and easy operation for the immobilization. The cross-linking method includes a method in which a cross-linking agent such as glutaraldehyde is used to form a cross-link between enzymes so as to combine the enzymes, and accordingly the enzymes are immobilized, and a method in which a matrix substance such as albumin is added to form a cross-link between the enzymes and the matrix substance, and accordingly the enzymes are immobilized along with the matrix substance.

However, since these cross-linking methods perform cross-linking through a covalent bond, activity of the enzymes often decreases, so that sufficient stability thereof cannot be obtained in many cases. Furthermore, because a way of cross-linking is different for each enzyme, and many reactions and refining operations are necessary for an immobilizing operation, the methods have problems that they are complicated, cost high, and have low versatility.

Moreover, in the enzyme electrodes using these cross-linking methods, the transmission and diffusion of a substance is prone to be poor because the cross-link makes a space between the enzymes small. Accordingly, the enzyme electrodes have problems having low sensitivity, poor repeatability, and a shorter operating life.

In the carrier binding method, enzymes are not easily desorbed since the method immobilizes the enzymes directly to resin or the like by a covalent bond. However, there are problems that its immobilizing operation is complicated, the enzymes are decomposed by proteolytic enzymes, and the steric structures of the enzymes change according to change of an external environment.

In the entrapment method such as the immobilization-by-entrapment method and the microcapsule method, since the size of a gel lattice or the size of an inter-capsule does not match the sizes of enzymes in general, the enzymes cannot be fixed firmly for the immobilization in the gel lattice or the capsule, so that the enzymes leak out and are deactivated. Furthermore, the entrapment method has little effect on preventing the change of the steric structures of the enzymes which is caused by the change of the external environment since structure stability of the gel lattice or the capsule is not sufficient.

Therefore, the enzyme electrode using the carrier binding method or the entrapment method lacks the stability. It is because the steric structures of the enzymes change according to the change of the external environment, and the activity of the immobilized enzymes decreases as time passes even during measurement. Accordingly, the enzyme sensor using the enzyme electrode also has problems having poor repeatability and a shorter operating life.

As the physical adsorption method, for example, a method in which mesoporous materials are formed systematically on a two-dimensional substrate, and then enzymes are immobilized in the mesoporous materials by physical adsorption is proposed. (For example, refer to patent documents 1 and 2 mentioned below.)

Document Z.H. Dai et. al., Bioelectrochemistry 70 (2007) 250-256, describes an enzyme electrode comprising an electrode, a mesoporous silica material on the electrode and an enzyme immobilised in the pores of the mesoporous material.

However, because the sizes of small cavities (inside diameters of small cavities) of the mesoporous material do not match the sizes of the enzymes (diameters of the enzymes) in general, the method for immobilizing the enzymes in the mesoporous material cannot fix the enzymes firmly for the immobilization in the small cavities. Therefore, problems that the activity of the enzymes decreases, the steric structures of the enzymes change according to the change of the external environment, and the like arise.

Hence, the enzyme electrode using the method for immobilizing the enzymes in the mesoporous material lacks the stability, and accordingly, the enzyme sensor using the enzyme electrode also has problems having poor repeatability and a shorter operating life.
Patent document 1: Japanese Unexamined Patent Publication No. 2002-346999
Patent document 2: Japanese Unexamined Patent Publication No. 2005-061961

### Disclosure of the Invention

### The Problems to be Solved by the Invention

That is to say, as described above, the enzyme electrodes using conventional methods of the immobilization and the enzyme sensors using the enzyme electrodes have problems such as lacking stability, and having low sensitivity, poor repeatability, and a shorter operating life because the enzymes are deactivated, the steric structures of the enzymes change according to the change of the external environment, the enzymes leak out, and the like.

Moreover, from the perspective of realization of a safe, secure and comfortable society, for example, a detecting technology and a detecting sensor to continuously monitor dwelling environmental pollutants such as formaldehyde and toluene, explosives such as trinitrotoluene (TNT) powder, narcotics such as cocaine and heroin, and the like at high speed and high sensitivity have been requested in recent years. Because these substances exist in a gaseous phase or a liquid phase at a very low concentration, high detection sensitivity which enables detection at a sub-ppb level is required to detect the substances. In addition, in order to enable continuous monitoring, a sensor having high stability and a longer operating life is desirable. That is to say, it is strongly desired to develop the enzyme sensor having excellent sensitivity, response speed, and stability as well as a longer operating life.

It is an object of the present invention to provide an enzyme electrode having excellent sensitivity, excellent stability, and a longer operating life, and an enzyme sensor using the enzyme electrode.

### Means for Solving the Problems

In order to achieve the above object, the invention is defined in claim 1. Preferred embodiments are defined in the dependent claims.

The embodiments and/or examples disclosed in the following description which are not covered by the appended claims are considered as not being part of the present invention.

### Effects of the Invention

According to the present disclosure, in the enzyme electrode and the enzyme sensor using the enzyme electrode, the enzyme electrode includes an electrode, a mesoporous silica material provided on the electrode, and an enzyme immobilized in small cavities of the mesoporous silica material, and the sizes of the small cavities are set to be 0.5 to 2.0 times the sizes of the enzymes.

Therefore, the enzymes can be fixed firmly for immobilization in the small cavities of the mesoporous silica material. As a result, the steric structures of the enzymes are prevented from changing, so that the enzyme electrode having excellent stability and a longer operating life and the enzyme sensor using the enzyme electrode can be provided.

In addition, the mesoporous silica material is porous and has a very large specific surface. Hence, comparing to the case of using a carrier which has a smaller specific surface than that of the mesoporous silica material, the enzymes can be immobilized with more adsorption at a higher concentration by using the mesoporous silica material as the carrier. Moreover, by fixing the enzymes firmly for the immobilization in the small cavities of the mesoporous silica material, the state where the enzymes are properly dispersed can be maintained, so that deactivation of the enzymes caused by aggregation thereof and the like can be prevented. That is to say, the enzyme electrode having excellent sensitivity and the enzyme sensor using the enzyme electrode can be provided. It is because the enzymes can be immobilized with more adsorption at a higher concentration, and deactivation of the enzymes caused by aggregation thereof and the like can be prevented by using, as the carrier, the mesoporous silica material of which the sizes of the small cavities are set to be 0.5 to 2.0 times the sizes of the enzymes.

### Best Mode for Carrying out the Invention

In the following, the best mode of the enzyme electrode and the enzyme sensor using the enzyme electrode of the present invention will be described in detail with reference to the figures. The scope of the invention is not limited to the shown examples.

An enzyme sensor 100 of the present invention is, for example, a sensor to detect a target substance by an electrochemical measurement using an enzyme electrode 1.

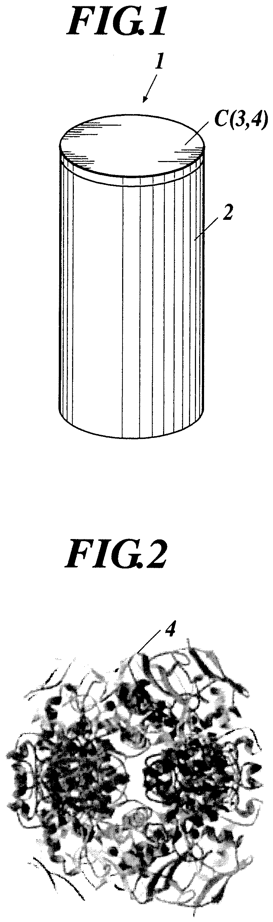

The enzyme electrode 1 of the present invention includes, for example, as shown in FIG. 1, an electrode 2, and an enzyme protein complex C formed with a mesoporous silica material 3 provided on the electrode 2 and enzymes 4 immobilized in small cavities of the mesoporous silica material 3.

The enzymes 4 are, for example, oxidation-reduction enzymes.

However, the enzymes 4 are not limited to the oxidation-reduction enzymes, and as long as they are enzymes (enzyme proteins), the enzymes 4 can be optionally chosen. For example, a hydrolytic enzyme, a transfer enzyme, an isomerizing enzyme, or the like may be used.

Moreover, the enzymes 4 may be natural enzyme molecules or enzyme fragments including active sites. The natural enzyme molecules or the enzyme fragments including the active sites may be extracted from animals, plants, or microorganisms, may be cut the extracted one by request, or may be synthesized by gene engineering or chemical engineering.

To be specific, as the oxidation-reduction enzymes, glucose oxidase, lactate oxidase, cholesterol oxidase, alcohol oxidase, formaldehyde oxidase, sorbitol oxidase, fructose oxidase, sarcosine oxidase, fructosyl amine oxidase, pyruvic acid oxidase, xanthine oxidase, ascorbic acid oxidase, sarcosine oxidase, choline oxidase, amine oxidase, glucose dehydrogenase, lactate dehydrogenase, cholesterol dehydrogenase, alcohol dehydrogenase, formaldehyde dehydrogenase, sorbitol dehydrogenase, fructose dehydrogenase, hydroxybutyric acid dehydrogenase, glycerol dehydrogenase, glutamate dehydrogenase, pyruvic acid dehydrogenase, malic acid dehydrogenase, glutamic acid dehydrogenase, catalase, peroxidase, and uricase can be used, for example. Other than these, cholesterol esterase, creatininase, creatinase, DNA polymerase, and mutants of these enzymes can be used, for example.

As the hydrolytic enzyme, protease, lipase, amylase, invertase, maltase, β-galactosidase, lysozyme, urease, esterase, nuclease, and phosphatase can be used, for example.

As the transfer enzyme, acyltransferase, kinase, and aminotransferase can be used, for example.

As the isomerizing enzyme, racemase, phosphoglycerate phosphomutase, and glucose-6-phosphate isomerase can be used, for example.

The enzymes 4 forming the enzyme protein complex C may be composed of one kind of, or two or more kinds of enzymes.

More specifically, the enzymes 4 forming the enzyme protein complex C may be composed of one kind of enzymes, two or more kinds of enzymes whose molecule weights and/or sizes (diameters) are almost the same, or two or more kinds of enzymes whose molecule weights and/or sizes (diameters) are different. Furthermore, when the enzymes 4 forming the enzyme protein complex C are composed of two or more kinds of enzymes, the enzymes 4 may act on the same kinds of specific substances (substrates), on different kinds of specific substances, or on the same and different kinds of specific substances.

Moreover, when the enzymes 4 forming the enzyme protein complex C are composed of two or more kinds of enzymes, the enzymes 4 may be immobilized in different or the same small cavities of the mesoporous silica material 3.

In particular, when the enzymes 4 forming the enzyme protein complex C are composed of two or more kinds of enzymes, and the enzymes 4 act on different kinds of specific substances, the enzyme sensor 100 can detect the different kinds of specific substances (two or more kinds of specific substances) simultaneously.

The mesoporous silica material 3 can be composed of metal oxide such as silicic acid and alumina, complex oxide of silicic acid and another kind of metal, or the like.

For example, to form the mesoporous silica material 3 composed of silicic acid, layered silicate such as kanemite, alkoxysilane, or silica gel, water glass, sodium silicate, or the like can be preferably used.

More specifically, the mesoporous silica material 3 is formed, for example, by producing a surfactant-inorganic composite in which an inorganic skeleton is formed around a micelle of the surfactant through a mixed reaction of the inorganic material with the surfactant, and thereafter by removing the surfactant through calcination at 400°C to 600°C, extraction using an organic solvent, or the like. Consequently, the mesoporous silica material 3 has mesoporous small cavities in the inorganic skeleton, the shapes of the small cavities being the same as the micelle of the surfactant.

When a silicon containing compound such as silicic acid is used as a starting material to produce the mesoporous silica material 3, for example, the small cavities thereof can be produced by forming layered silicate such as kanemite, inserting a micelle between layers thereof, combining the layers not having the micelle in between by silicate molecules, and removing the micelle thereafter.

When a silicon containing substance such as water glass is used as the starting material to produce the mesoporous silica material 3, for example, the small cavities thereof can be produced by assembling silicate molecules around a micelle, polymerizing the silicate molecules in order to form silica, and removing the micelle thereafter. In this case, usually the micelle is columnar, and as a result, columnar small cavities are produced in the mesoporous silica material 3.

The mesoporous silica material 3 can control the inside diameters of the small cavities by changing the lengths of alkyl chains of the surfactant so as to vary the diameters of the micelles at a step of production. Furthermore, addition of relatively hydrophobic molecules such as trimethylbenzene or tripropylbenzene along with the surfactant can swell the micelles, and accordingly produce the small cavities having larger diameters.

The sizes (diameters) of the small cavities of the mesoporous silica material 3 are determined based on the sizes (diameters) of the enzymes 4 to be immobilized. That is to say, the mesoporous silica material 3 having the small cavities whose sizes are 0.5 to 2.0 times the sizes of the enzymes 4 to be immobilized can be obtained by producing the mesoporous silica material 3 using the surfactant with the micelles whose sizes (diameters of the micelles) are 0.5 to 2.0 times the sizes of the enzymes 4.

The mesoporous silica material 3 is powdery, granulated, layered, bulk, membranous, or the like.

The mesoporous silica material 3 may be produced individually as described above, or produced on the substrate or the electrode directly utilizing deposition by a spin coating or nucleus growth, or utilizing an external field such as photo-orientation, an electric field, a magnetic field, shear flow, or the like with the direction in addition to the sizes of the small cavities controlled.

The small cavities of the mesoporous silica material 3 may be oriented at random, or with the directivity thereof controlled like that of one-dimensional silica nanochannel assembly.

Publically known KSW, FSM, SBA, MCM, HOM, or the like in which the sizes of the small cavities are uniform and which has high porosity can be employed as the mesoporous silica material 3.

Furthermore, publically known CTAB-M, P123-M, F127-M, or the like in which the sizes of the small cavities are uniform and the directions of the small cavities (channels) go in one direction can be employed as the mesoporous silica material 3. To be more specific, CTAB-M, P123-M, F127-M, or the like is, for example, a membranous mesoporous silica material 3 filled with the mesoporous silica nanochannel assembly (one-dimensional silica nanochannel assembly) which is produced in cylindrical alumina small cavities using a surfactant as a template, and which has the same channel direction as the alumina small cavities.

The sizes of the small cavities of the mesoporous silica material 3 are set within the range where the steric structures of the enzymes 4 to be immobilized can be prevented from changing. Accordingly, the steric structures of the enzymes 4 to be immobilized can be maintained, and the enzymes 4 immobilized in the mesoporous silica material 3 can be stabilized.

To put it concretely, for example, the sizes of the small cavities of the mesoporous silica material 3 are preferably about 0.5 to 2.0 times, more preferably about 0.7 to 1.4 times, and most preferably almost equal to the sizes of the enzymes 4 (the enzyme molecules or the enzyme fragments including the active sites) to be immobilized. That is to say, the diameters of the small cavities (central small cavity diameters) of the mesoporous silica material 3 are preferably about 0.5 to 2.0 times, more preferably about 0.7 to 1.4 times, and most preferably almost equal to the diameters of the enzymes 4 to be immobilized. The specific central small cavity diameters are determined in relation to the diameters of the enzymes 4, and hence cannot be defined uniformly. However, the central small cavity diameters can be set at about 1 to 50 nm.

When the enzymes form a polymer, the sizes (diameters) of the enzymes 4 to be immobilized can be the size (diameter) of the polymer. Here, the polymer is a compound produced by a bond of two or more enzymes (proteins) directly or via low weight molecules such as water. The bond includes a covalent bond, an ionic bond, a hydrogen bond, and a coordinate bond. However, the kinds of bonds are not limited to these in particular.

The specific surface area of the mesoporous silica material 3 is, for example, about 200 to 1,500 m².

The depths of the small cavities of the mesoporous silica material 3 are 2 nm or more. More specifically, the range of the depths is preferably 20 to 100 *µ*m, more preferably 50 to 500 nm, and most preferably 50 to 150 nm.

The pitches of the small cavities of the mesoporous silica material 3 are, when the pitch of the small cavities is defined as a distance between the centers of the small cavities, preferably 2 to 500 nm, more preferably 2 to 100 nm, and most preferably 2 to 50 nm.

By setting the sizes of the small cavities of the mesoporous silica material 3 to be about 0.5 to 2.0 times (more preferably about 0.7 to 1.4 times, and most preferably almost equal to) the sizes of the enzymes 4 to be immobilized, maintenance of the steric structures of the enzymes 4 to be immobilized becomes easy, so that the enzymes 4 immobilized in the mesoporous silica material 3 can be stabilized.

More specifically, when formaldehyde dehydrogenase shown in FIG. 2 is employed as the enzymes 4, for example, the diameter of formaldehyde dehydrogenase is about 8 nm. Therefore, the sizes of the small cavities of the mesoporous silica material 3 are set to be preferably about 0.5 to 2.0 times, more preferably about 0.7 to 1.4 times, and most preferably, as shown in FIG. 3, almost equal to the diameter of formaldehyde dehydrogenase. Consequently, as shown in FIG. 4, formaldehyde dehydrogenase adsorbs and is immobilized on inner walls of the small cavities of the mesoporous silica material 3 with the steric structure of formaldehyde dehydrogenase maintained, and forms the enzyme protein complex C.

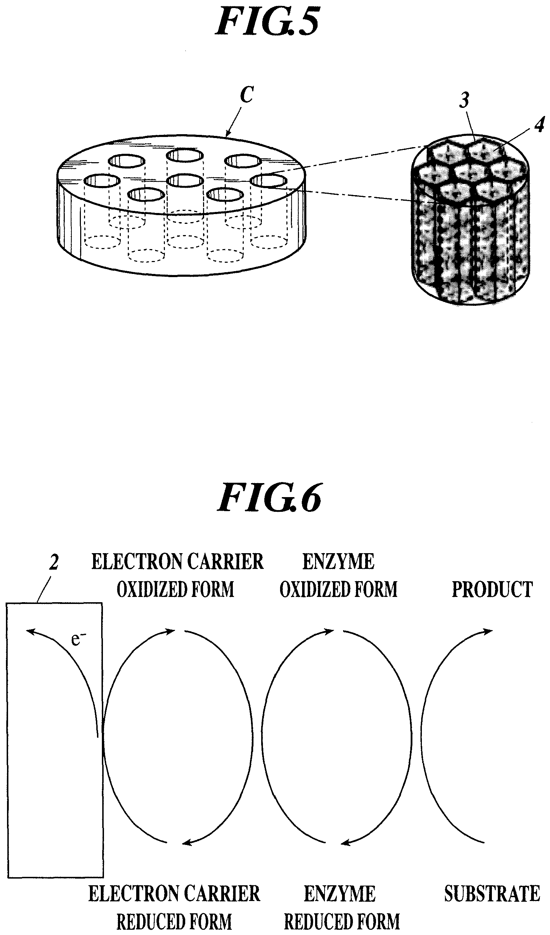

When the membranous mesoporous silica material 3 filled with the one-dimensional silica nanochannel assembly is used as the mesoporous silica material 3 as shown in FIG. 5, for example, the sizes of the small cavities are also set to be preferably about 0.5 to 2.0 times, more preferably about 0.7 to 1.4 times, and most preferably almost equal to the diameter of formaldehyde dehydrogenase. Consequently, formaldehyde dehydrogenase adsorbs and is immobilized on the inner walls of the small cavities (channels) of the mesoporous silica material 3 with the steric structure of formaldehyde dehydrogenase maintained, and forms the enzyme protein complex C.

Since the enzymes 4 are stably immobilized in the small cavities of the mesoporous silica material 3 in which the sizes of the small cavities are about 0.5 to 2.0 times the sizes of the enzymes 4, electron transfer between active centers in the enzyme molecules and the electrode 2 is difficult except for the case where a product is oxidized or reduced directly by the electrode 2. Also, in the case where the enzymes 4 are immobilized in the small cavities of the mesoporous silica material 3 in which the small cavities have high aspect ratios and the directivities thereof are controlled like the one-dimensional silica nanochannel assembly, a response is very slow even when the product is oxidized or reduced directly by the electrode 2.

Therefore, preferably an electron carrier to facilitate the transfer of the electron between the enzymes 4 and the electrode 2 should be introduced into the small cavities of the mesoporous silica material 3, for example.

Use of an oxygen electrode, a hydrogen peroxide electrode, or the like as the electrode 2 causes problems that only a sample with a low concentration can be measured because the concentration of dissolved oxygen limits a reaction, and the electrode has low selectivity because it is prone to be influenced by an oxidant such as ascorbic acid, for example. In these cases as well, use of the electron carrier along with the enzymes 4 is effective to expand a detection range and improve the selectivity.

To put it concretely, potassium ferricyanide, ferrocene, a ferrocene derivative, benzoquinone, a quinine derivative, and an osmium complex can be used as the electron carrier, for example.

It is also preferable to introduce a metal atom, a metal ion, a metal complex, dye, or the like (Fe²⁺, Mn²⁺, Cu²⁺, Zn²⁺, Co³⁺, for example) into the small cavities of the mesoporous silica material 3 as a coenzyme or a cofactor to catalyze the expression of the activity of the enzymes 4.

For example, in the case of a reaction which does not proceed easily by catalysis of amino acid side chains of the enzymes 4 such as a reaction via an unstable intermediate, a small organic molecule, the metal ion, or the metal complex which has a proper structure and a low molecular weight, and which is involved with the expression of the enzyme reaction is often used as the cofactor. Among the cofactors, the small organic molecule and the metal complex are called as coenzymes. In particular, in the case of using a coenzyme-dependent enzyme, the enzyme reaction can be efficiently performed by introducing the coenzyme into the small cavities of the mesoporous silica material 3.

The coenzyme can be suitably selected according to the kinds of the enzymes 4 (coenzyme-dependent enzymes). More specifically, a coenzyme 300 may be nicotinamide adenine dinucleotide (NAD⁺), nicotinamide adenine dinucleotide phosphate (NADP⁺), coenzyme I, coenzyme II, flavin mononucleotide (FMN), flavin adenine dinucleotide (FAD), lipoic acid, adenosine triphosphate (ATP), thiamin pyrophosphate (TPP), pyridoxal phosphate (PALP), tetrahydrofolic acid (THF, Coenzyme F), UDP glucose (UDPG), coenzyme A, coenzyme Q, biotin, coenzyme B₁₂ (cobalamin), S-adenosylmethionine, or a combination of two or more kinds of these, for example.

In the enzyme electrode 1 of the present invention, it is desired that either the enzyme carrier or the coenzyme, preferably both of them, be introduced into the small cavities of the mesoporous silica material 3, and be layered on and contacted with the enzyme electrode 2. Other than that, the electron carrier and/or the coenzyme may be dissolved and dispersed in an electrolytic solution, and disposed on a window for analysis of the electrode 2 by dropping or the like when the enzyme electrode 1 is in use, or only the electron carrier may be applied to a surface of the electrode 2 filmily, and the mesoporous silica material 3 in which the enzymes 4 and the coenzyme are immobilized in the small cavities may be immobilized thereon.

It is also preferable to introduce, for example, water molecules which are necessary for the expression of the activity of the enzymes 4 into the small cavities of the mesoporous silica material 3.

When the water molecules are introduced into and adsorbed onto the small cavities of the mesoporous silica material 3, the enzymes 4 can be prevented from drying and protected from a cause of deactivation since adsorbed water on silica does not evaporate so soon. Consequently, resistance to dryness of the enzyme electrode 1 improves, and accordingly long-term stability of the enzyme electrode 1 and the enzyme sensor 100 also improves.

In addition, it is also preferable to provide the enzyme electrode 1 with a permselective film.

Here, the permselective film is either a film which transmits only a specific substance selectively, or a film which does not transmit a specific substance selectively.

Employing the permselective film can improve the selectivity of the enzyme sensor 100 even when, for example, an enzyme having the wide substrate specificity is used as the enzymes 4. That is to say, when, for example, alcohol oxidase having the wide substrate specificity is used as the enzymes 4, although the alcohol oxidase responses to both alcohol and formaldehyde, not alcohol but formaldehyde is transmitted toward the enzyme electrode 1 even when there are alcohol and formaldehyde which is the target substance in a liquid or a gas by using a film which does not transmit alcohol, a polyallylamine (PAAM) film for example, as the permselective film, so that the selectivity of the enzyme sensor 100 can be improved. In this case, the permselective film should be provided such that the enzyme protein complex C is covered.

When, for example, the concentration of glucose is measured from the amount of hydrogen peroxide produced by the enzyme reaction; glucose + oxygen - (glucose oxidase) → gluconic acid + hydrogen peroxide, using a platinum electrode or the like, the selectivity of the enzyme sensor 100 can be improved by using the permselective film which transmits hydrogen peroxide but does not easily transmit an oxidant such as ascorbic acid because the measurement is easily influenced by the oxidant such as ascorbic acid. More specifically, a cellulose acetate film, a polypyrrole film, an ion exchange resin film, or the like can be used as the permselective film which selectively transmits hydrogen peroxide. In this case, the permselective film should be provided between the enzyme protein complex C and the electrode 2.

The permselective film may be a ceramic porous film which selectively filters a high-molecular-weight substance, an ionic conductive film which does not have a practical cavity such as zirconia and perovskites, or a carrier transport film in which a complex reaction is utilized.

As a material, a polymer film, a metal film, a ceramic film, and an ionic conductive film can be used, for example. As the metal film, a palladium film can be used, for example. As the ceramic film, a zeolite film and a silica film can be used, for example. As the polymer film, a polydimethylsiloxane film, a polytrimethylsilylpropyne film, a fluorine-containing acrylic resin film, a cellulose acetate film, a polyester film, a polyimide film, a fluoropolymer film, a polyethylene film, a polystyrene film, a polyethylene terephthalate film, a vinylidene chloride film, a polyethylene oxide film, a polyphosphazen film, a polyacetylene film, a polyaniline film, a polyvinyl alcohol film, an ethylene vinyl alcohol copolymer, a carbon film, a facilitated transport film, or the like, or a combination of these films can be used, for example.

One kind or two or more kinds of permselective films may be provided with the enzyme electrode 1.

The permselevtive film may be attached to the enzyme electrode 1 via a mechanic system utilizing an O-ring or the like, or directly attached to the electrode 2 or a surface of the enzyme protein complex C by a method such as a dip coating method, a spin coating method, a potting method, a vapor deposition method, a sputtering method, a plasma treatment, or the like. The method can be appropriately selected as needed.

Furthermore, by using these methods, a plurality of the permselective films can be stratified consecutively so as to form a multilayer film. This multilayer film may include the enzyme immobilized film (enzyme protein complex C) . More specifically, the enzyme protein complexes C can have a layer structure with the permselective films being inserted therebetween, for example.

Moreover, in order to secure adhesion of the permselective film and stability of the sensor, the permselective film can be covered with a protective film or a predetermined film, and physically held down thereby, or the like.

For the immobilization of the mesoporous silica material 3 on the electrode 2, the mesoporous silica material 3 may be formed directly on the electrode 2 by the dip coating method, the spin coating method method, the potting method, the vapor deposition method, the sputtering method, the plasma treatment, or the like, may be adhered onto the electrode 2 by synthetic resin, photo cross-linking resin, or the like, or may be immobilized on the electrode 2 by using polyethylene glycol or the like as a binder. Also, the mesoporous silica material 3 may be produced directly on the substrate or the electrode by utilizing the deposition by the spin coating or the nucleus growth, by utilizing the external field such as the photo-orientation, the electric field, the magnetic field, the shear flow, or the like, or by producing the mesoporous silica material 3 in the alumina small cavities formed by anodic oxidation, or the like.

Moreover, the mesoporous silica material 3 may also be immobilized on the electrode 2 by utilizing a nylon net filter 1a having a network structure of a few microns, or the like, even during measurement in a solution, for preventing a leakage and the like of the mesoporous silica material 3 (enzyme protein complex C) in which the enzymes 4 are immobilized, for example.

As an electrode system of the enzyme sensor 100, a two-electrode system, the electrodes being a working electrode and a counter electrode, or a three-electrode system, the electrodes being the working electrode, the counter electrode, and a reference electrode, may be employed.

As the electrode 2 which is the working electrode, a precious metal such as gold, platinum, copper, and aluminum, SnO₂, In₂O₃, WO₃, TiO₂, graphite, glassy carbon, or the like can be used.

As the counter electrode, gold in the two-electrode system, and silver or other metals in the three-electrode system can be used.

As the reference electrode, a calomel electrode, silver-silver chloride, or the like can be used.

The electrode used in an electrolysis cell is, as shown in FIGs. 1, 8, and 9, an example of the working electrode (electrode 2), the counter electrode, and the reference electrode. However, these electrodes are not particularly limited in sizes, shapes, and configurations.

More specifically, these electrodes may be large electrodes used in commercial electrolysis cells, conductance cells, or the like, may be disc electrodes, rotating ring-disc electrodes, fiber electrodes, or the like, or may be micro-electrodes (disc electrodes, cylindrical electrodes, strip electrodes, arranged strip electrodes, arranged cylindrical electrodes, ring electrodes, spherical electrodes, comb electrodes, pair-electrodes, or the like) made by a publically known fine processing technology such as photolithography.

These electrodes can be provided on predetermined insulating substrates.

More specifically, each electrode can be formed on the insulating substrate by a publicly known method such as a screen printing method, the vapor deposition method, or the sputtering method. All of the electrodes may be formed on the same substrate to make an integral enzyme sensor 100, may be formed on a plurality of the substrates to make the enzyme sensor 100, or may be formed as individual electrodes to make the enzyme electrode 100.

As the insulating substrate, ceramics, glass, plastics, paper, a biodegradable material (for example, polyester produced by microorganisms), or the like can be used.

The methods for making the enzyme electrode 1 are not particularly limited. For example, the enzyme electrode 1 may be made by immobilizing the enzyme protein complex C on the electrode 2 after forming the enzyme protein complex C by immobilizing the enzymes 4 in the mesoporous silica material 3 through the dipping method in which a solution containing the enzymes 4 is dropped into the mesoporous silica material 3, through an immersion method in which the mesoporous silica material 3 is immersed in the solution containing the enzymes 4, or the like, may be made by immobilizing the enzymes 4 in the mesoporous silica material 3 through the dipping method, the immersion method, or the like after immobilizing the mesoporous silica material 3 on the electrode 2, or may be made by introducing the enzymes 4 into the mesoporous silica material 3 through utilization of the external field such as the electric field. Consequently, the enzymes 4 can be immobilized in the silica mesoporus 3 maintaining a higher order structure and the activity thereof.

In addition, a publicly known enzyme immobilizing method (for example, an immobilizing method using a conductive polymer, glutaraldehyde, photo cross-linking resin, or the like) can be used in combination with the above-mentioned methods as needed.

As a sensing method by the enzyme sensor 100 using the enzyme electrode 1, an electrochemical measurement may be used, for example. That is to say, a publicly known measurement such as chronoamperometry, coulometry, or cyclic voltammetry to measure an oxidation current or a reduction current can be employed. As a measuring system, a disposable system, a batch system, a flow injection system, or the like can be used.

Furthermore, as the electrochemical measurement, a photo detecting measurement which detects a color change of a chromogen caused by oxidation or reduction can be also employed. More specifically, for example, in a glucose sensor, two kinds of enzymes, glucose oxidase and peroxidase, are immobilized, and by an enzyme reaction; glucose + O₂ - (glucose oxidase) → gluconic acid +H₂O₂, and thereafter, H₂O₂ + chromogen - (peroxidase) → reddish purple color, hydrogen peroxide and a chromogen (for example, a mixture of 4-aminoantipyrine and N-ethyl-N(2-hydroxyl-3-sulfopropyl)-m-toluidine) are produced or consumed. The glucose sensor can measure the concentration of glucose by detecting the color change of hydrogen peroxide and the chromogen being reddish purple by means of peroxidase as a catalyst.

It is preferable that a measuring instrument body in which the enzyme sensor 100 using the enzyme electrode 1 of the present invention is installed for the measurement have a function of transmitting data to a computer with or without a wire so as to check a measurement value on a real-time basis, for example. It is also desirable that the body be configured to be capable of installing a plurality of kinds of enzyme sensors 100 and have a function of measuring the results detected by the plurality of kinds of enzyme sensors 100 simultaneously, and comparing and examining the data.

Next, a principle of measuring the concentration of the target substance in the sample at high speed and high sensitivity through the electrochemical measurement by using the enzyme sensor 100 using the enzyme electrode 1 of the present invention is described referring to FIG. 6.

In FIG. 6, oxidase (enzymes 4) is immobilized in the small cavities of the mesoporous silica material 3, for example. The immobilized oxidase oxidizes the substrate which is the target substance in the sample by selective catalysis so as to be reductase. Thereafter, by setting the working electrode (electrode 2) to be positive and applying a voltage between the working electrode and the reference electrode, reductase transmits the electron (e⁻) to the working electrode directly or indirectly via the electron carrier, and returns to oxitase. At that time, an electric current which re-oxidizes reductase or a reduced electron carrier flows between the working electrode and the reference electrode. Since a value of the electric current is proportional to an enzyme reaction rate, namely the substrate concentration in the sample, the concentration of the target substance in the sample can be calculated by measuring the value of the electric current.

In the following, the present invention will be described by means of concrete examples.

### First Example

### (1) Synthesis of Mesoporous Silica Material 3

In a first example, first, a mesoporous silica material 3 was synthesized.

More specifically, 271.59 g of water glass No. land 828.41 g of water were mixed, and heated at 80°C thereafter. Separately, 80 g of docosyltrimethylammoniumchloride (DTMA-C1) was added to 1 L of water at 70°C. After the solution became completely transparent, 70 mL of triisopropyl benzene was added to the solution, and the solution was stirred hard by a homomixer for 30 minutes. This emulsified solution was immediately added to the water glass solution and stirred for another 5 minutes. 2-normal hydrochloric acid was added to this solution taking around one hour, and stirred at pH 8.5 for around three hours. After suction filtration of the solution was performed, dispersion into heated water at 70°C and the filtration of the solution were repeated. A white powdery mesoporous silica material 3 was obtained by calcination of the solution for six hours in an electric furnace at 550°C after drying the solution for three days at 45°C.

The structure of the white powder was measured by a powder X-ray diffractometer (Rigaku RAD-B), and the diameters of the small cavities, the surface area, and the total capacity of the small cavities thereof were measured by a nitrogen adsorption apparatus. As a result, the white powder was confirmed as the powder of the mesoporous silica material having the structure in which two-dimensional hexagonal small cavities are disposed, the average small cavity diameter being about 8.2 nm. The obtained mesoporous silica material 3 may be referred as a large diameter FSM hereinafter.

### (2) Formation of Enzyme Protein Complex C

Next, an enzyme protein complex C was formed by immobilizing enzymes 4 in the mesoporous silica material 3. As the enzymes 4, formaldehyde dehydrogenase was used, for example.

More specifically, 50 mg of powder of the large diameter FSM and 5 mL (molar concentration of formaldehyde dehydrogenase: 4.2 mg/mL) of a formaldehyde dehydrogenase solution (phosphate buffer pH 6.9) were mixed, and stirred gently for 24 hours at 4°C by a rotator (about 100 rpm) . Thereafter, the mixed solution was centrifuged at 7,000 rpm for three minutes and a solid was collected therefrom. For three times, the mixed solution was cleaned by 5 mL of deionized water and centrifuged under the same condition as described above. Consequently, the enzyme protein complex C formed with formaldehyde dehydrogenase and the large diameter FSM was obtained.

When adsorption of formaldehyde dehydrogenase in the enzyme protein complex C was measured by using a supernatant liquid obtained by the centrifugation, the adsorption on the large diameter FSM was 88 mg/g.

### (3) Activity Test of Immobilized Enzymes

Next, activity of the enzymes 4 immobilized in the mesoporous silica material 3 was measured.

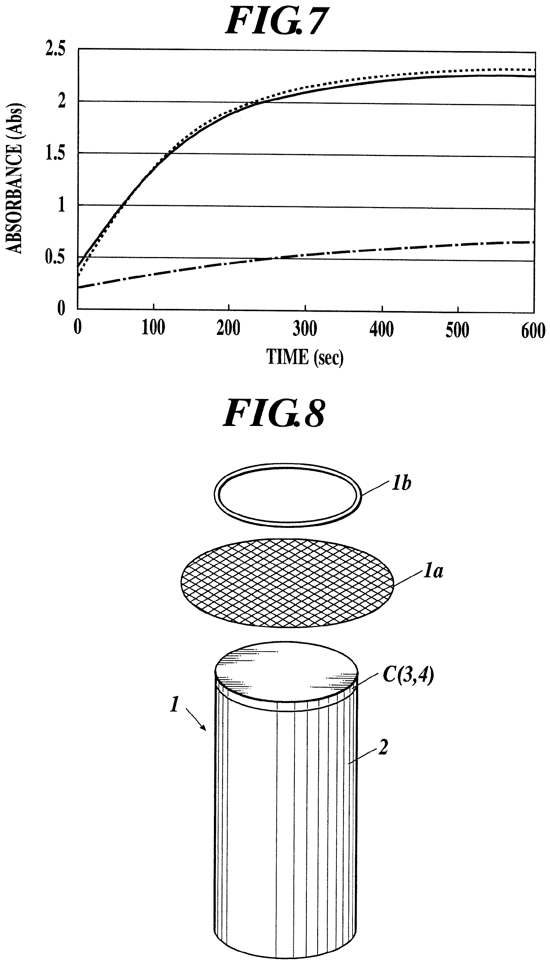

More specifically, 10 mg of the enzyme protein complex C, 1.2 mg of NAD⁺ as a coenzyme, and 300 *µ*L of an aqueous formaldehyde solution (concentration: 0.3%) which was a substrate were added to 2.7 mL of phosphate buffer (pH = 7.41) and reacted at 25°C. Because absorption was observed at 340 nm characteristic to NADH which was produced at the same time that formaldehyde was oxidized, 340 nm was used as an indicator. For comparison, the same measurement was carried out for a free enzyme (free formaldehyde dehydrogenase) and an enzyme cross-linked by glutaraldehyde (formaldehyde dehydrogenase cross-linked by glutaraldehyde) . The amounts of the enzymes 4 immobilized in the large diameter FSM, the free enzyme, and the enzyme cross-linked by glutaraldehyde, all of which were used for the measurement, were set to be the same. The result is shown in FIG. 7.

In FIG. 7, the horizontal axis indicates a reaction time and the vertical axis indicates an absorbance at 340 nm at which the absorption was observed when NADH was produced. The solid line, the broken line, and the long-dashed-short-dashed line indicate the results of the enzymes 4 immobilized in the large diameter FSM, the free enzyme, and the enzyme cross-linked by glutaraldehyde, respectively.

As shown in FIG. 7, the enzyme cross-linked by glutaraldehyde greatly declined its activity compared with the free enzyme. On the other hand, the enzymes 4 immobilized in the large diameter FSM proceeded the reaction at the same level as the free enzyme. Consequently, it was confirmed that the enzymes 4 immobilized in the small cavities of the large diameter FSM did not denature, and the activity thereof was maintained.

### (4) Enzyme Electrode 1 Making

Next, an enzyme electrode 1 was made by using the enzyme protein complex C.

More specifically, as an electrode 2 (working electrode), a columnar glassy carbon electrode of 3.2 mm in diameter and 0.1 cm² in area was prepared. Then, 5 mg of the enzyme protein complex C was applied and immobilized to the surface of the carbon electrode. Furthermore, the enzyme protein complex C was adhered and immobilized onto the working electrode to prevent a leakage thereof and the like by a nylon net filter 1a (bore diameter: 11 *µ*m) and an O-ring 1b as shown in FIG. 8, for example. In this manner, the enzyme electrode 1 which includes the electrode 2, and the enzyme protein complex C formed with the mesoporous silica material 3 (large diameter FSM) and the enzymes 4 (formaldehyde dehydrogenase) was made. The enzyme electrode 1 was stored at 4°C until the time of use.

### (5) Electrochemical Measurement

Next, a target substance was detected through an electrochemical measurement by an enzyme sensor 100 using the enzyme electrode 1.

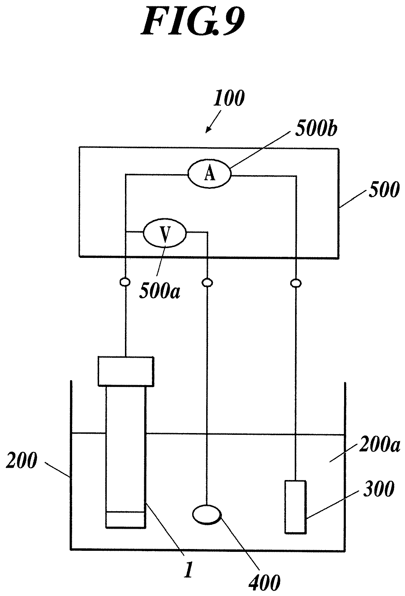

More specifically, as shown in FIG. 9 for example, the enzyme sensor 100 was made by using the enzyme electrode 1. The enzyme sensor 100 includes, for example, the working electrode (enzyme electrode 1), a counter electrode 300, and a reference electrode 400, all of which are in a reactor 200 and connected to a lead, and as an electrolytic solution 200a, contains 10.0 mL of the phosphate buffer (pH = 7.41) in which 1 mM nicotinamide adenine dinucleotide (NAD⁺) and 1 mM quinine are dissolved. A potentiostats 500 to measure a value of an electric current generated at the enzyme electrode 1 is connected to the lead which is connected to the working electrode (enzyme electrode 1), the counter electrode 300, and the reference electrode 400. The potentiostat 500 has, for example, a constant voltmeter 500a and an electric current measuring apparatus 500b.

When the substrate is added into the electrolytic solution under the condition where a voltage having an electric potential higher than that of the reference electrode 400 by 350 mV is applied to the working electrode (enzyme electrode 1) of the enzyme sensor 100, the substrate, the enzymes 4, a coenzyme, and the like react. At the time, because an electron is transferred between the electrode 2 and the enzymes 4 via an electron carrier, an electric current flows into the enzyme electrode 1. The concentration of the target substance can be detected by measuring the electric current as an oxidized current with the potentiostat 500.

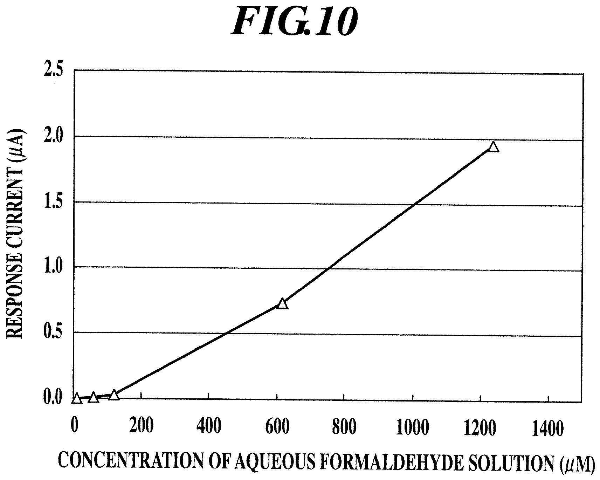

In the enzyme sensor 100, the voltage having the electric potential higher than that of the reference electrode 400 by 350 mV was applied to the working electrode (enzyme electrode 1) and aged. After aging, the aqueous formaldehyde solution (concentration: 0.6 *µ*M to 1,200 *µ*M) was added as the substrate to the buffer under the condition where the electric potential was applied to the electrode, and then an output response current was measured. The result is shown in FIG. 10.

In FIG. 10, the horizontal axis indicates the concentration of the aqueous formaldehyde solution and the vertical axis indicates the output response current.

As shown in FIG. 10, when the concentration of the aqueous formaldehyde solution was in a range of 0 to 1,200 *µ*M, the electric current which flowed increased nearly linearly as the concentration of the substrate increased. Also, high output and the good linearity were seen in the low concentration range. Consequently, it was confirmed that the enzyme reaction was performed well without the enzyme deactivated. In particular, it was confirmed that the enzyme sensor 100 had very high detection sensitivity, had 1.2 *µ*M of a detection limit, and enabled high-sensitive detection equivalent to the sub-ppb level detection assuming that the condition would be vapor-liquid equilibrium.

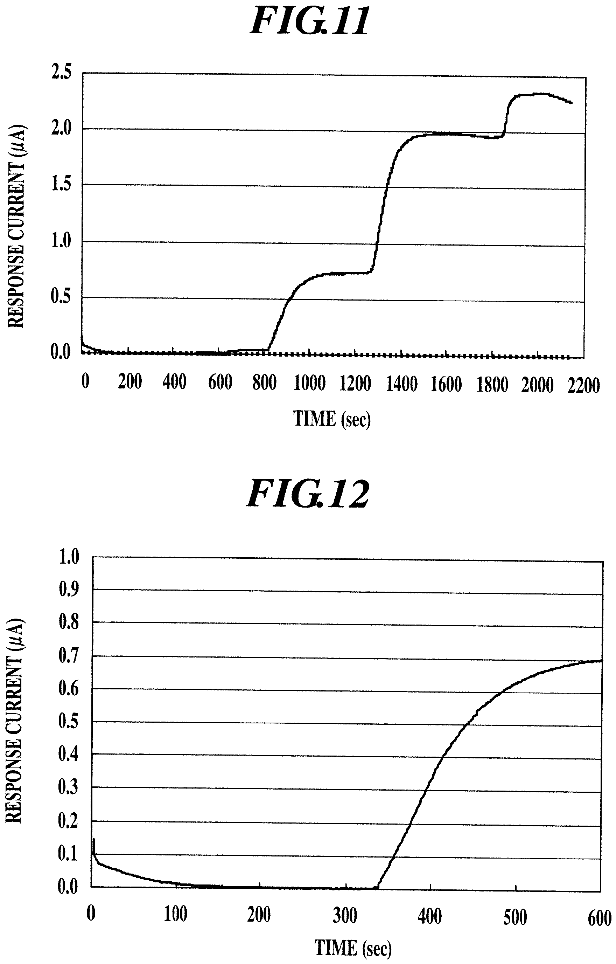

Effectiveness of introduction of the electron carrier into the small cavities of the mesoporous silica material 3 was examined. The result is shown in FIG. 11.

FIG. 11 shows the result of a measurement of the response current which was output when seven aqueous formaldehyde solutions, each of the solutions having a different concentration (concentrations: 0.6 *µ*M, 1.2 *µ*M, 6 *µ*M, 12 *µ* M, 60 *µ*M, 120 *µ*M, 600 *µ*M, and 1,200 *µ*M), were added consecutively to the buffer under the condition where the electric potential was applied to the electrode. The horizontal axis indicates the response time, and the vertical axis indicates the response current. The solid line indicates the result of the case where the electron carrier was introduced, and the broken line indicates the result of the case where the electron carrier was not introduced.

As shown in FIG. 11, in the case where the electron carrier was not introduced, the response current was not output at all. On the other hand, in the case where the electron carrier was introduced, the response current was output sufficiently even when the concentration of the aqueous formaldehyde solution was low, and the more the concentration of the aqueous formaldehyde solution increased, the more the response current was output. Consequently, it was confirmed that the introduction of the electron carrier into the small cavities of the mesoporous silica material 3 was effective.

Furthermore, the response time was measured when the aqueous formaldehyde solution (concentration: 600 *µ*M) was added to the buffer under the condition where the electric potential was applied to the electrode of the enzyme sensor 100. The result is shown in FIG. 12.

In FIG. 12, the horizontal axis indicates the response time and the vertical axis indicates the response current.

As shown in FIG. 12, a 90% response time was about 120 seconds. Consequently, the enzyme sensor was confirmed to have a high response speed.

Here, the 90% response time is a time period from a time the substrate (aqueous formaldehyde solution) was added to a time the current value became 90% of a final steady current value.

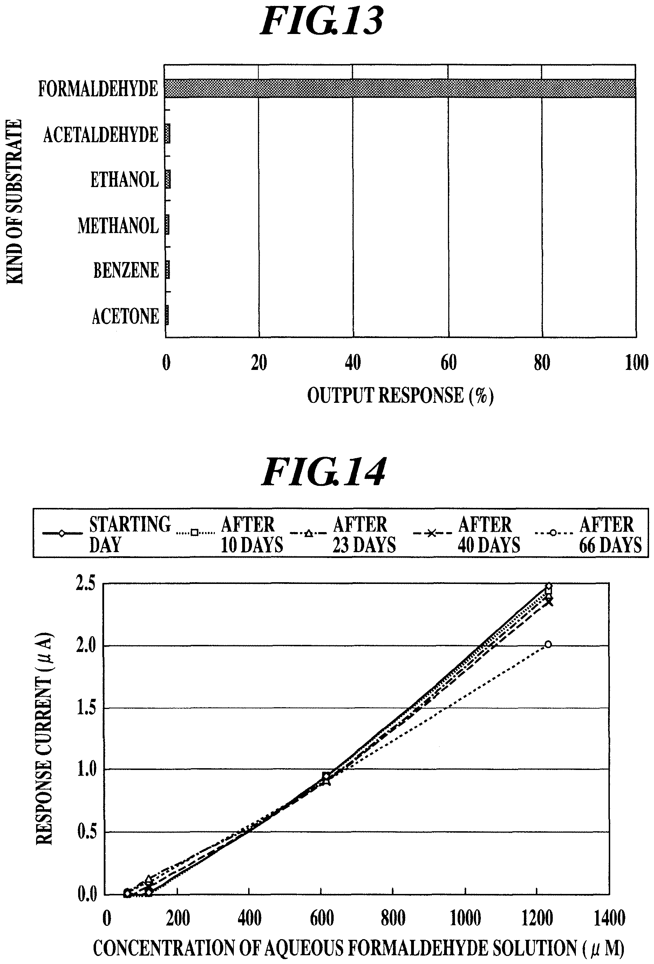

Selectivity of the enzyme sensor 100 was examined. The result is shown in FIG. 13.

Acetaldehyde, ethanol, methanol, benzene, and acetone were used as the substance other than formaldehyde. In FIG. 13, the horizontal axis indicates the output response of the substance solutions (concentrations: 6.2 *µ*M) regarding the output response of the aqueous formaldehyde solution (concentration: 6.2 *µ*M) as 100%.

As shown in FIG. 13, it was confirmed that the enzyme sensor 100 had very high selectivity based on substrate specificity of the enzymes 4 (formaldehyde dehydrogenase).

Repeatability of the enzyme sensor 100 was examined. The result is shown in FIG. 14.

More specifically, the enzyme electrode 1 was stored in the buffer placed in a refrigerator at 4°C. After 10, 23, 40, and 66 days, the aqueous formaldehyde solution (concentration: 0.6 *µ*M to 1,200 *µ*M) was added as the substrate to the buffer under the condition where the electric potential was applied to the electrode, and the output response current was measured.

In FIG. 14, the horizontal axis indicates the concentration of the added aqueous formaldehyde solution, and the vertical axis indicates the response current. The lines plotted with rhombuses (◇), quadrilaterals (□), triangles (Δ), crosses (×), and circles (○) indicate the results on the starting day (day 0), after 10 days, after 23 days, after 40 days, and after 66 days, respectively.

As shown in FIG. 14, the enzyme sensor 100 declined the output slightly, but obtained almost the same result after 10, 23, 40 and 66 days compared with the starting day. Consequently, it was confirmed that the sensor had good repeatability and excellent linearity. As a result, it was confirmed that the enzyme sensor 100 declined the activity of the enzymes 4 only slightly, had good repeatability, and enabled a long-term measurement.

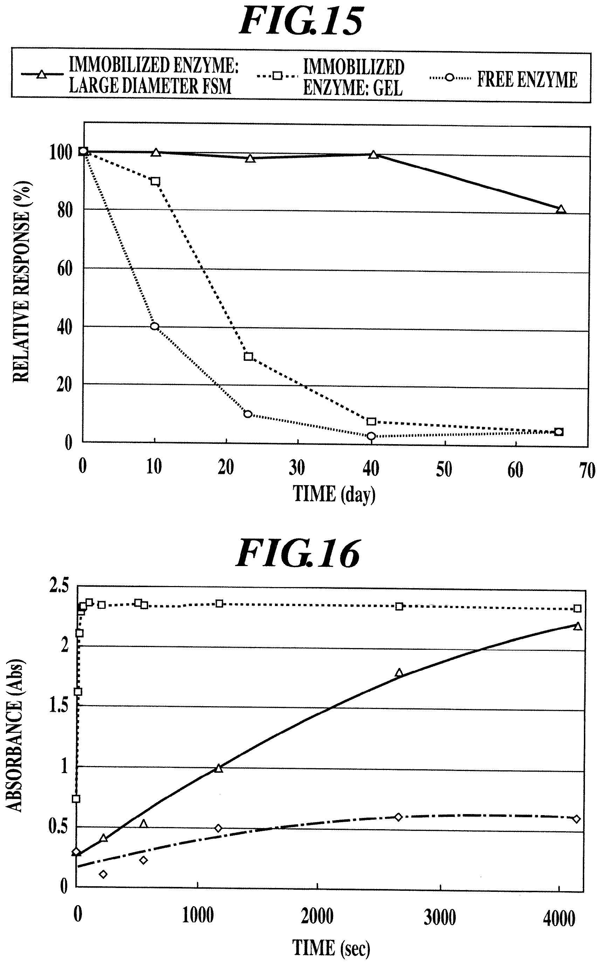

Effectiveness of immobilization of the enzymes 4 in the mesoporous silica material 3 was examined. The result is shown in FIG. 15.

More specifically, the same measurement as the measurement whose result is shown in FIG. 14 was carried out for the enzyme electrode including agarose gel in which formaldehyde dehydrogenase was immobilized, and for the free enzyme. The free enzyme was measured under the condition where formaldehyde dehydrogenase was not provided with the electrode but included in the electrolytic solution 200a in the reactor 200.

In FIG. 15, the horizontal axis indicates the reaction time, and the vertical axis indicates a relative response (namely, the response current regarding the response current on the starting day as 100%) . The lines plotted with triangles (Δ), quadrilaterals (□), and circles (○) indicate the results of the electrode including the mesoporous silica material 3 with the enzymes 4 (formaldehyde dehydrogenase) immobilized, the enzyme electrode including agarose gel with formaldehyde dehydrogenase immobilized, and the free enzyme, respectively. That is to say, the line plotted with triangles (Δ) indicates the result calculated from the result obtained by the aqueous formaldehyde solution having a concentration of 1,200 *µ*M shown in FIG. 14.

As shown in FIG. 15, the output response currents of the enzyme electrode including agarose gel with formaldehyde dehydrogenase immobilized and the free enzyme declined over time and did not change almost at all after 40 days and thereafter . Consequently, it was confirmed that the enzyme activity thereof was lost. On the other hand, the enzyme electrode 1 of the present invention output almost the same amount of the response current after 10, 23, and 40 days as that on the starting day. After 66 days, although the response became about 80% of that on the starting day, the enzyme electrode 1 output a significantly large amount of the response current compared with the enzyme electrode including agarose gel with formaldehyde dehydrogenase immobilized, and the free enzyme.

### (6) Principle of Electrochemical Measurement

In the following, a principle of the electrochemical measurement in the first example will be described.

The enzymes 4 (formaldehyde dehydrogenase) immobilized in the small cavities of the mesoporous silica material 3 (large diameter FSM) on the electrode 2 become reductase by oxidizing formaldehyde which is the target substance (substrate) in a sample. When formaldehyde dehydrogenase which is a coenzyme-dependent enzyme reacts with formaldehyde which is a substrate to produce formic acid, formaldehyde dehydrogenase is converted into a reduced coenzyme (NADH) by NAD⁺ which is an oxidized coenzyme receiving hydrogen. Thereafter, the working electrode (enzyme electrode 1) is turned to be positive and a voltage is applied between the working electrode and the reference electrode. Then, NADH moves the hydrogen and conveys transfer of the produced electron to the working electrode (electrode 2) via the electron carrier, and the reduced enzyme returns to be the oxidized enzyme. At the time, the electric current to re-oxidize the reduced enzyme or the reduced electron carrier flows between the working electrode and the reference electrode, and a value of the electric current is measured.

In the first example, although the enzymes 4 (formaldehyde dehydrogenase of about 8 nm in diameter) are immobilized in the small cavities of the mesoporous silica material 3 (large diameter FSM having an average small cavity diameter of about 8.2 nm), the sizes of the small cavities being almost equal to the sizes of the enzymes 4 , a high-speed response is achieved by transferring the electron between the enzymes 4 and the electrode 2 via the electron carrier.

Furthermore, the transmission and diffusion of the substance is good. It is because the enzymes 4 are adsorbed onto the mesoporous silica material 3 which is porous and has a very large surface area, and the state where the enzymes 4 are properly dispersed is maintained. Also, high-sensitive detection is achieved by utilizing concentration action of the mesoporous silica material 3 on the target substance.

Moreover, the immobilization and remarkable stabilization of the enzymes 4 are achieved by retaining the steric structures of the enzymes 4 by inner walls of the small cavities of the mesoporous silica material 3.

Consequently, very high-speed and high-sensitive detection of the target substance became available, and accordingly the enzyme sensor 100 which works at high speed and high sensitivity, and has a longer operating life, excellent repeatability, and excellent stability was made.

### Second Example

### (1) Synthesis of Mesoporous Silica Material 3

In a second example, first, the membranous mesoporous silica material 3 filled with one-dimensional silica nanochannel assembly was synthesized.

More specifically, 1.0 g of PEG-P123 copolymer, 20 mL of ethanol, 2 mL of water, and 100 *µ*L of concentrated hydrochloric acid were mixed, and thereafter refluxed for one hour at 60°C being stirred. Furthermore, 2.1 g of tetraethylorthosilicate (TEOS) was added to the solution, and the solution was refluxed for two hours at 60°C. Then, 4 mL of this solution was extracted and dropped into a porous anodized aluminum film (diameter: 47 mm, thickness: 0.6 *µ*M, and small cavity diameter: 0.1 *µ*M). Then, drying was performed at normal temperature in a desiccator for 20 minutes after vacuum filtration. The membranous mesoporous silica material 3 was obtained by calcination in the electric furnace at 500°C for five hours.

The membranous mesoporous silica material 3 was measured by a transmission electron microscope (TEM). As a result, it was confirmed that silica nanochannels having an average small cavity diameter of about 8.2 nm were filled in the alumina small cavities of 100 nm in average small cavity diameter along the walls of the alumina small cavities. The obtained membranous mesoporous silica material 3 may be referred as a large diameter mesoporous film hereinafter.

### (2) Formation of Enzyme Protein Complex C

Next, the enzyme protein complex C was formed by immobilizing the enzymes 4 in the mesoporous silica material 3. As the enzymes 4, formaldehyde dehydrogenase was used, for example.

More specifically, 2.5 mL (molar concentration: 1.2 mg/mL) of the formaldehyde dehydrogenase solution (phosphate buffer pH 7.4) was dropped into 50 mg of the large diameter mesoporous film and kept still in a refrigerator (3°C) over night. Thereafter, the large diameter mesoporous film was cleaned by 5 mL of deionized water for three times continuously so as to wash away the enzyme from its surface. Consequently, the enzyme protein complex C formed with formaldehyde dehydrogenase and the large diameter mesoporous film was obtained.

When adsorption of formaldehyde dehydrogenase in the enzyme protein complex C was measured by using a thermogravimetric analyzer (TG-DTA), the adsorption on the large diameter mesoporous film was 2.7 mg/g.

### (3) Activity Test of Immobilized Enzyme

Next, the activity of the enzymes 4 immobilized in the mesoporous silica material 3 was measured.

More specifically, the enzyme protein complex C which was powdered, 12 mg of NAD⁺ as the coenzyme, and 300 *µ*L of the aqueous formaldehyde solution (concentration: 0.3%) which was the substrate were added to 3 . 0 mL of the phosphate buffer (pH = 7.41) and reacted at 25°C. Then, 600 *µ*L of supernatant liquid of the reacted solution was extracted and mixed with 400 *µ*L of the phosphate buffer. Absorption of the mixed liquid at 340 nm was measured. For comparison, the same measurement was carried out for the free enzyme (free formaldehyde dehydrogenase) and the enzyme cross-linked by glutaraldehyde (formaldehyde dehydrogenase cross-linked by glutaraldehyde). The amounts of the enzymes 4 immobilized in the large diameter mesoporous film, the free enzyme, and the enzyme cross-linked by glutaraldehyde, all of which were used for the measurement, were set to be the same. The result is shown in FIG. 16.

In FIG. 16, the horizontal axis indicates the reaction time and the vertical axis indicates the absorbance at 340 nm at which the absorption was observed when NADH was produced. The solid line, the broken line, and the long-dashed-short-dashed line indicate the results of the enzymes 4 immobilized in the large diameter mesoporous film, the free enzyme, and the enzyme cross-linked by glutaraldehyde, respectively.

As shown in FIG. 16, the enzyme cross-linked by glutaraldehyde greatly declined its activity compared with the free enzyme. On the other hand, although the enzymes 4 immobilized in the large diameter mesoporous film had a slow reaction speed owing to the limit of the diffusion of the coenzyme to the enzymes 4 immobilized in the columnar small cavities having large aspect ratios, the enzymes 4 maintained the activity at the same level as the free enzyme. Consequently, it was confirmed that the enzymes 4 immobilized in the small cavities of the large diameter mesoporous film did not denature and the activity thereof was maintained.

### (4) Enzyme Electrode 1 Making

Next, the enzyme electrode 1 was made by using the enzyme protein complex C.

More specifically, as the electrode 2 (working electrode), the columnar glassy carbon electrode of 3.2 mm in diameter and 0.1 cm² in area was prepared. The enzyme protein complex C cut into pieces of 3 mm square was adhered and immobilized to the surface of the carbon electrode, which was the working electrode, for example, by the nylon net filter 1a (bore diameter: 11 *µ* m) and the O-ring 1b as shown in FIG. 8. In this manner, the enzyme electrode 1 which includes the electrode 2 and the enzyme protein complex C was made. The enzyme protein complex C was formed with the mesoporous silica material 3 (large diameter mesoporous film) which had columnar small cavities nearly perpendicularly produced to the electrode 2, and the enzymes 4 (formaldehyde dehydrogenase). The enzyme electrode 1 was stored at 4°C until the time of use.

### (5) Electrochemical Measurement

Next, the target substance was detected through the electrochemical measurement by the enzyme sensor 100 using the enzyme electrode 1.

More specifically, as shown in FIG. 9 for example, the enzyme sensor 100 was made by using the enzyme electrode 1. The voltage having the electric potential higher than that of the reference electrode 400 by 350 mV was applied to the working electrode (enzyme electrode 1) of the enzyme sensor 100. Accordingly, an electric current flowed into the working electrode (enzyme electrode 1), and the current was measured by the potentiostat 500.

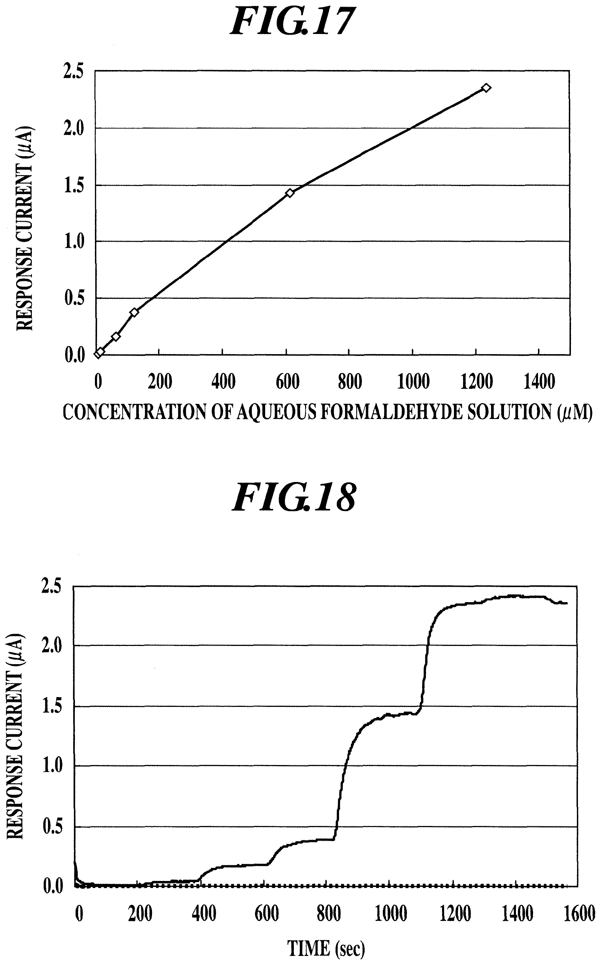

In the sensor 100, the voltage having the electric potential higher than that of the reference electrode 400 by 350 mV was applied to the working electrode (enzyme electrode 1) and aged. After aging, the aqueous formaldehyde solution (concentration: 0.6 *µ*M to 1,200 *µ*M) was added as the substrate to the buffer under the condition where the electric potential was applied to the electrode, and then the output response current was measured. The result is shown in FIG. 17.

In FIG. 17, the horizontal axis indicates the concentration of the aqueous formaldehyde solution, and the vertical axis indicates the output response current.

As shown in FIG. 17, when the concentration of the aqueous formaldehyde solution was in a range of 0 to 1,200 *µ*M, high output and good linearity were seen. Consequently, it was confirmed that the enzyme reaction was performed well without the enzyme deactivated. Also, it was confirmed that the enzyme sensor 100 had very high detection sensitivity, had 1.2 *µ*M of the detection limit, and enabled the high-sensitive detection equivalent to the sub-ppb level detection assuming that the condition would be vapor-liquid equilibrium.

The effectiveness of the introduction of the electron carrier into the small cavities of the mesoporous silica material 3 was examined. The result is shown in FIG. 18.

FIG. 18 shows the result of the measurement of the response current which was output when seven aqueous formaldehyde solutions, each of the solutions having a different concentration (concentrations: 0.6 *µ*M, 1.2 *µ*M, 6 *µ*M, 12 *µ* M, 60 *µ*M, 120 *µ*M, 600 *µ*M and 1,200 *µ*M), were added consecutively to the buffer under the condition where the electric potential was applied to the electrode. The horizontal axis indicates the response time, and the vertical axis indicates the response current. The solid line indicates the result of the case where the electron carrier was introduced, and the broken line indicates the result of the case where the electron carrier was not introduced.

As shown in FIG. 18, in the case where the electron carrier was not introduced, the response current was not output at all. On the other hand, in the case where the electron carrier was introduced, the response current was output sufficiently even when the concentration of the aqueous formaldehyde solution was low, and the more the concentration of the aqueous formaldehyde solution increased, the more the response current was output. Consequently, it was confirmed that the introduction of the electron carrier into the small cavities of the mesoporous silica material 3 was effective.

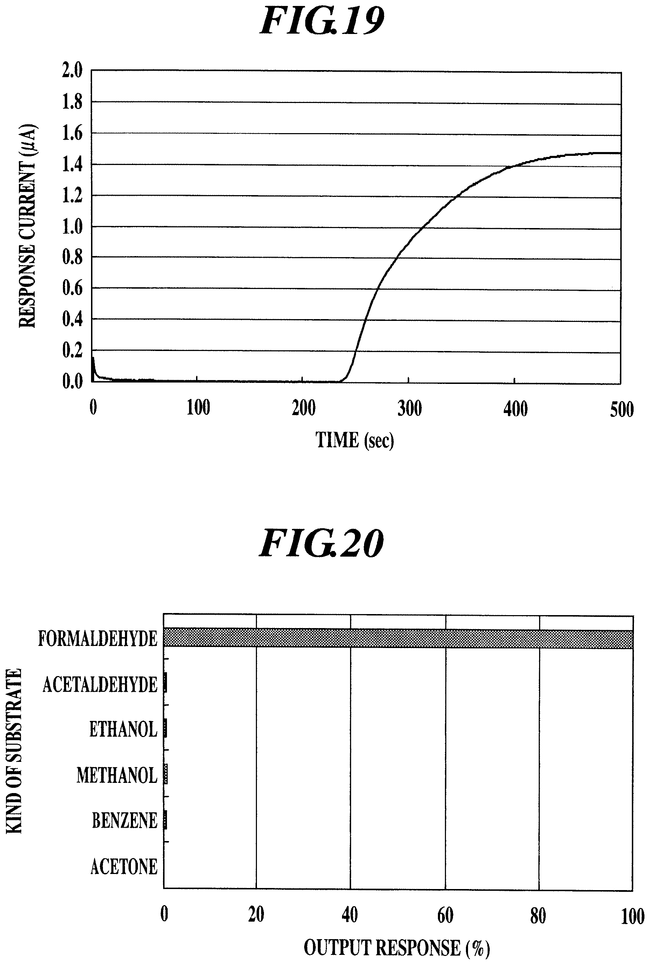

Furthermore, the response time was measured when the aqueous formaldehyde solution (concentration: 600 *µ*M) was added to the buffer under the condition where the electric potential was applied to the electrode of the enzyme sensor 100. The result is shown in FIG. 19.

In FIG. 19, the horizontal axis indicates the response time, and the vertical axis indicates the response current.

As shown in FIG. 19, the 90% response time, the time period from the time the substrate (aqueous formaldehyde solution) was added to the time the current value became 90% of the final steady current value, was about 120 seconds. Consequently, the enzyme sensor was confirmed to have a high response speed.

Moreover, the response speed of the enzyme sensor 100 was confirmed to be very stable and highly repeatable by repeated measurement. It is because the thickness of the film of the mesoporous silica material 3 is controlled very accurately with the length of the nanochannels of the large diameter mesoporous film being determined by the thickness of the anodized aluminum film, which is the template, although the response speed is in inverse proportion to the square of the film thickness.

The selectivity of the enzyme sensor 100 was examined. The result is shown in FIG. 20.

Acetaldehyde, ethanol, methanol, benzene, and acetone were used as the substance other than formaldehyde. In FIG. 20, the horizontal axis indicates the output response of the substance solutions (concentrations: 6.2 *µ*M) regarding the output response of the aqueous formaldehyde solution (concentration: 6.2 *µ*M) as 100%.

As shown in FIG. 20, it was confirmed that the enzyme sensor 100 had very high selectivity based on the substrate specificity of the enzymes 4 (formaldehyde dehydrogenase).

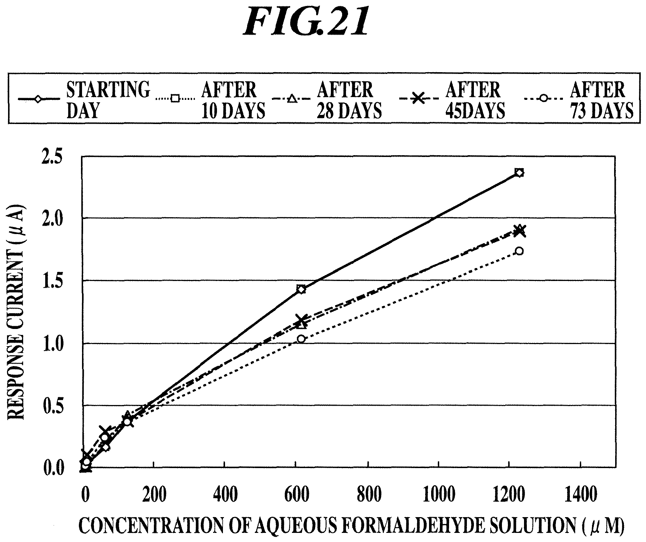

The repeatability of the enzyme sensor 100 was examined. The result is shown in FIG. 21.

More specifically, the enzyme electrode 1 was stored in the buffer placed in a refrigerator at 4°C. After 10, 28, 45, and 73 days, the aqueous formaldehyde solution (concentration: 0.6 *µ*M to 1,200 *µ*M) was added as the substrate to the buffer under the condition where the electric potential was applied to the electrode, and the output response current was measured.

In FIG. 21, the horizontal axis indicates the concentration of the added aqueous formaldehyde solution, and the vertical axis indicates the response current. The lines plotted with rhombuses (◇), quadrilaterals (□), triangles (Δ), crosses (×), and circles (○) indicate the results on the starting day (day 0), after 10 days, after 28 days, after 45 days, and after 73 days, respectively.

As shown in FIG. 21, the enzyme sensor 100 declined the output slightly, but obtained almost the same result after 10, 28, 45 and 73 days compared with the starting day. Consequently, it was confirmed that the sensor had good repeatability and excellent linearity. As a result, it was confirmed that the enzyme sensor 100 declined the activity of the enzymes 4 only slightly, had good repeatability, and enabled a long-term measurement.

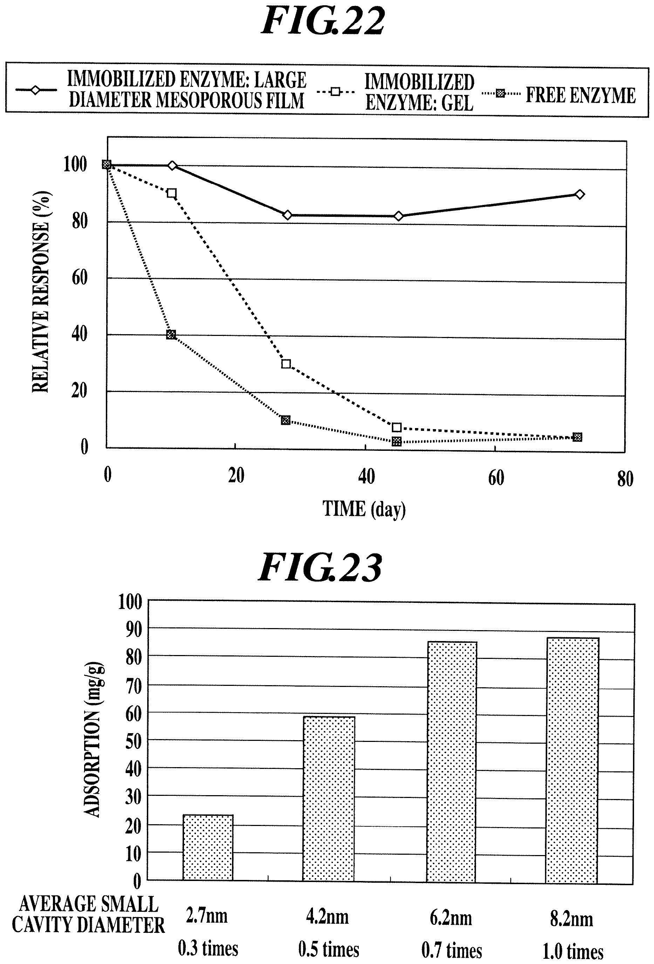

The effectiveness of the immobilization of the enzymes 4 in the mesoporous silica material 3 was examined. The result is shown in FIG. 22.

More specifically, the same measurement as the measurement whose result is shown in FIG. 22 was carried out for the enzyme electrode including agarose gel in which formaldehyde dehydrogenase was immobilized, and for the free enzyme. The free enzyme was measured under the condition where formaldehyde dehydrogenase was not provided with the electrode but included in the electrolytic solution 200a in the reactor 200.

In FIG. 22, the horizontal axis indicates the reaction time, and the vertical axis indicates the relative response (namely, the response current regarding the response current on the starting day as 100%) . The lines plotted with rhombuses (◊), quadrilaterals (□), and circles (○) indicate the results of the electrode including the mesoporous silica material 3 with the enzymes 4 (formaldehyde dehydrogenase) immobilized, the enzyme electrode including agarose gel with formaldehyde dehydrogenase immobilized, and the free enzyme, respectively. That is to say, the line plotted with rhombuses (◊) indicates the result calculated from the result obtained by the aqueous formaldehyde solution having a concentration of 1,200 *µ*M shown in FIG. 21.

As shown in FIG. 21, the output response currents of the enzyme electrode including agarose gel with formaldehyde dehydrogenase immobilized and of the free enzyme declined over time and did not change almost at all after 45 days and thereafter. Consequently, it was confirmed that the enzyme activity thereof was lost. On the other hand, the enzyme electrode 1 of the present invention output almost the same amount of the response current after 10 days as that on the starting day. After 28, 45, and 73 days, although the response became about 80% of that on the starting day, the enzyme electrode 1 output a significantly large amount of the response current compared with the enzyme electrode including agarose gel with formaldehyde dehydrogenase immobilized, and the free enzyme.

### (6) Principle of Electrochemical Measurement

The principle of the electrochemical measurement in the second example is the same as that in the first example.

In the second example, since silica nanochannels are produced perpendicularly to the surface of the electrode 2, transmission of a substance is good. In addition, a high-speed and high-sensitive response is achieved by transferring the electron between the active centers of the enzymes 4 and the electrode 2 via the electron carrier although a diffusion velocity is limited because of the high aspect ratios of the silica nanochannels, the ratios being about 1,000 to 10,000.

Consequently, very high-speed and high-sensitive detection of the target substance became available, and accordingly the enzyme sensor 100 which works at high speed and high sensitivity, and has a longer operating life, excellent repeatability, and excellent stability was made.

### Third Example

### (1) Synthesis of Mesoporous Silica Material 3

In a third example, first, the mesoporous silica materials 3 and the membranous mesoporous silica materials 3 filled with one-dimensional silica nanochannel assembly were synthesized.

More specifically, the white powdery mesoporous silica materials 3 having average small cavity diameters of about 2.7 nm, 4.2 nm, 6.2 nm and 8.2 nm were obtained by a similar method to that of the first example or the like. The obtained mesoporous silica materials 3 may be referred as FSMs hereinafter.

Also, the membranous mesoporous silica materials 3 having average small cavity diameters of about 8.2 nm, 12.2 nm, 17.8 nm, and 98.4 nm were obtained by a similar method to that of the second example or the like. The obtained membranous mesoporous silica materials 3 may be referred as mesoporous films hereinafter.

### (2) Formation of Enzyme Protein Complex C

Next, the enzyme protein complexes C were formed by immobilizing the enzymes 4 in the mesoporous silica materials 3. As the enzymes 4, formaldehyde dehydrogenase was used, for example.

More specifically, the enzyme protein complexes C formed with formaldehyde dehydrogenase and the FSMs were obtained by a similar method to that of the first example or the like.

Also, the enzyme protein complexes C formed with formaldehyde dehydrogenase and the mesoporous films were obtained by a similar method to that of the second example or the like.

Here, the sizes of the small cavities of the FSMs having average small cavity diameters of about 2.7 nm, 4.2 nm, 6.2 nm, and 8.2 nm are about 0.3, 0.5, 0.7, and 1.0 times the size of formaldehyde dehydrogenase, respectively. The sizes of the small cavities of the mesoporous films having average small cavity diameters of about 8.2 nm, 12.2 nm, 17.8 nm, and 98.4 nm are about 1.0, 1.5, 2.2, and 12.3 times the size of formaldehyde dehydrogenase, respectively.

By similar methods to the first and second examples or the like, the adsorption in the enzyme protein complexes C was measured. The result is shown in FIG. 23.

FIG. 23 shows the adsorption of formaldehyde dehydrogenase on the FSMs having average small cavity diameters of about 2.7 nm, 4.2 nm, 6.2 nm and 8.2 nm, respectively.

As shown in FIG. 23, in the case where the sizes of the small cavities of the mesoporous silica material 3 were about 1.0 times the sizes of the enzymes 4 (namely, the case where the average small cavity diameter was about 8.2 nm), the adsorption was the largest. The adsorption of this case may be referred as the maximum adsorption hereinafter.

When the sizes of the small cavities of the mesoporous silica material 3 were about 0.7 times the sizes of the enzymes 4 (namely, when the average small cavity diameter was about 6.2 nm), the adsorption was smaller than, but almost equal to the maximum adsorption. When the sizes of the small cavities of the mesoporous silica material 3 were about 0.5 times the sizes of the enzymes 4 (namely, when the average small cavity diameter was about 4.2 nm), the adsorption was about 70% of the maximum adsorption. These indicate that a small difference between the sizes of the small cavities of the mesoporous silica material 3 and the sizes of the enzymes 4 is acceptable owing to flexibility of the enzymes 4.

On the other hand, when the sizes of the small cavities of the mesoporous silica material 3 were about 0.3 times the sizes of the enzymes 4 (namely, when the average small cavity diameter was about 2.7 nm), the adsorption was 25% or less of the maximum adsorption.

Consequently, it was confirmed that the large adsorption could be obtained by setting the sizes of the small cavities of the mesoporous silica material 3 to be 0.5 times or more (more preferably about 0.7 times or more, and most preferably almost equal to) the sizes of the enzymes 4 to be immobilized, so that the high-sensitive enzyme sensor 100 could be made.

### (3) Enzyme Electrode 1 Making

Next, the enzyme electrodes 1 were made by using the enzyme protein complexes C.

More specifically, the enzyme electrode 1 including the electrode 2, and the enzyme protein complex C formed with the mesoporous silica material 3 (FSM) and the enzymes 4 (formaldehyde dehydrogenase) was made by a similar method to that of the first example or the like.

Also, the enzyme electrode 1 including the electrode 2, and the enzyme protein complex C formed with the membranous mesoporous silica material 3 (mesoporous film) having a plurality of columnar cavities (channels) which were nearly perpendicularly produced to the electrode 2 and the enzymes 4 (formaldehyde dehydrogenase) was made by a similar method to that of the second example or the like. The enzyme electrodes 1 were stored at 4°C until the time of use.

### (4) Electrochemical Measurement

Next, the target substance was detected through the electrochemical measurement by the enzyme sensors 100 using the enzyme electrodes 1.

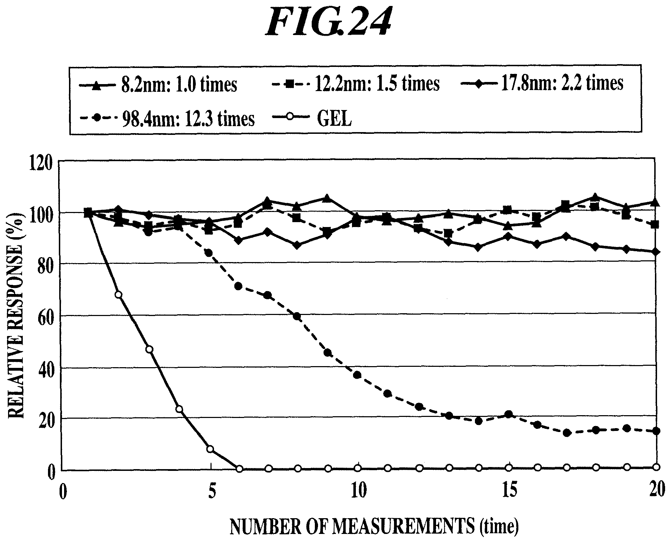

First, stability of repeated measurement was examined. The result is shown in FIG. 24.

More specifically, with regard to the enzyme electrodes 1 including the mesoporous silica materials 3 (mesoporous films) having average small cavity diameters of about 8.2 nm, 12.2 nm, 17.8 nm, and 98.4 nm in which formaldehyde dehydrogenase was immobilized, and the enzyme electrode including agarose gel in which formaldehyde dehydrogenase was immobilized, the aqueous formaldehyde solution (concentration: 600 *µ*M) was added as the substrate to the buffer under the condition where the electric potential was applied to the electrodes. The output response current thereby was measured. The measurement was repeated. Every time the measurement was completed, the enzyme immobilized electrodes were cleaned three times with distilled water, and then moved to the next time of the measurement.

In FIG. 24, the horizontal axis indicates the number of measurements, and the vertical axis indicates the relative response (namely, the response current regarding the response current at the first time of the measurement as 100%). The lines plotted with black triangles (▲), black quadrilaterals (■), black rhombuses (◆), and black circles (●) indicate the results of the electrodes including the mesoporous silica materials 3 having average small cavity diameters of about 8.2 nm, 12.2 nm, 17.8 nm, and 98.4 nm in which the enzymes 4 are immobilized, respectively. The line plotted with white circles (○) indicates the result of the electrode including agarose gel in which the enzymes 4 are immobilized.

As shown in FIG. 24, in the case where the sizes of the small cavities of the mesoporous silica material 3 were about 1.0 times the sizes of the enzymes 4 (namely, the case where the average small cavity diameter was about 8.2 nm), the response did not decline even when the number of times the measurement was repeated increased. In the case where the sizes of the small cavities of the mesoporous silica material 3 were about 1.5 times the sizes of the enzymes 4 (namely, the case where the average small cavity diameter was about 12.2 nm), the response did not decline almost at all even when the number of times the measurement was repeated increased. The degree of the decline of the response was greater than, but almost equal to the case where the sizes of the small cavities of the mesoporous silica material 3 were about 1.0 times the sizes of the enzymes 4. In the case where the sizes of the small cavities of the mesoporous silica material 3 were about 2.2 times the sizes of the enzymes 4 (namely, the case where the average small cavity diameter was about 17.8 nm), the response did not decline almost at all even when the number of times the measurement was repeated increased. At the 20^{th} time of the measurement, the response was still 80% or more of the response at the first time of the measurement. That is to say, in the cases where the sizes of the small cavities of the mesoporous silica materials 3 were about 1.0, 1.5, and 2.2 times the sizes of the enzymes 4 (namely, the cases where the average small cavity diameters were about 8.2 nm, 12.2 nm, and 17.8 nm), the response did not decline almost at all even when the number of times the measurement was repeated increased. This indicates that since the enzymes 4 are firmly fixed for the immobilization in the small cavities of the mesoporous silica material 3 by setting the sizes of the small cavities to fit the enzymes 4, the steric structures thereof are prevented from changing and is stabilized.

On the other hand, in the case where the sizes of the small cavities of the mesoporous silica material 3 were about 12.3 times the sizes of the enzymes 4 (namely, the case where the average small cavity diameter was about 98.4 nm), the output response current declined as the number of times the measurement was repeated increased. At the tenth time of the measurement and thereafter, the response became 40% or less compared with that at the first time of the measurement. Furthermore, in the case where the sizes of the small cavities were about 100 times the sizes of the enzymes 4 (namely, the case of agarose gel), the response current declined as the number of times the measurement was repeated increased. At the sixth time of the measurement and thereafter, the response current was not output. The cause is considered elution of the enzymes 4 by cleaning and the like, the enzymes 4 having not been firmly fixed for the immobilization in the small cavities of the mesoporous silica material 3 by setting the sizes of the small cavities to fit the enzymes 4.

As a result, it was confirmed that the enzyme sensor 100 with excellent stability and repeatability could be made by setting the sizes of the small cavities of the mesoporous silica material 3 to be about 2.0 times or less (preferably about 1.4 times or less, and most preferably almost equal to) the sizes of the enzymes 4 to be immobilized.

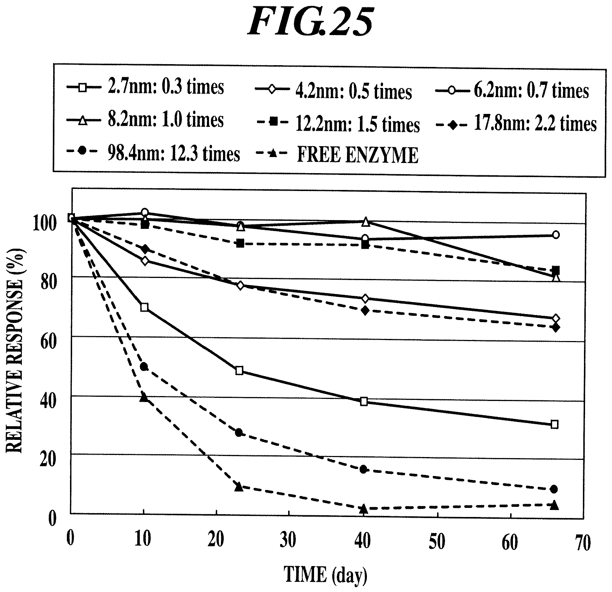

The repeatability of the enzyme sensor 100 was examined by a similar method to that of the first example or the like. The result is shown in FIG. 25.

More specifically, the same measurement as the measurement whose result is shown in FIG. 14 was carried out for the enzyme electrodes 1 including the mesoporous silica materials 3 (FSMs) having average small cavity diameters of about 2.7 nm, 4.2 nm, 6.2 nm, and 8.2 nm in which formaldehyde dehydrogenase was immobilized, the electrodes 1 including the mesoporous silica materials 3 (mesoporous films) having average small cavity diameters of about 12.2 nm, 17.8 nm, and 98.4 nm in which formaldehyde dehydrogenase was immobilized, and the free enzyme. The free enzyme was measured under the condition where formaldehyde dehydrogenase was not provided with the electrode but included in the electrolytic solution 200a in the reactor 200.

In FIG. 25, the horizontal axis indicates the reaction time, and the vertical axis indicates the relative response (namely, the response current regarding the response current on the starting day as 100%). The lines plotted with white quadrilaterals (□), white rhombuses (◊), white circles (○), white triangles (Δ), black quadrilaterals (■), black rhombuses (◆), and black circles (●) indicate the results of the electrodes including the mesoporous silica materials 3 having average small cavity diameters of about 2.7 nm, 4.2 nm, 6.2 nm, 8.2 nm, 12.2 nm, 17.8 nm, and 98 .4 nm in which the enzymes 4 are immobilized, respectively, and the line plotted with black triangles (▲) indicates the result of the free enzyme. That is to say, the line with white triangles (Δ) indicates the result calculated from the result obtained by the aqueous formaldehyde solution having a concentration of 1,200 *µ*M shown in FIG. 14.

As shown in FIG. 25, in the cases where the sizes of the small cavities of the mesoporous silica materials 3 were about 0.7, 1.0, and 1.5 times the sizes of the enzymes 4 (namely, the cases where the average small cavity diameters were about 6.2 nm, 8.2 nm, and 12.2 nm), the response did not decline almost at all even when time passed, and was still 80% or more response after 66 days compared with that on the starting day. Furthermore, in the cases where the sizes of the small cavities of the mesoporous silica materials 3 were about 0.5 and 2.2 times the sizes of the enzymes 4 (namely, the cases where the average small cavity diameters were about 4.2 nm and 17.8 nm), the response declined as time passed, but still had 60% or more response after 66 days compared with that on the starting day. This indicates that since the enzymes 4 are firmly fixed for the immobilization in the small cavities of the mesoporous silica material 3 by setting the sizes of the small cavities to fit the enzymes 4, the steric structures thereof are prevented from changing and are stabilized. It also indicates that a small difference between the sizes of the small cavities of the mesoporous silica material 3 and the sizes of the enzymes 4 is acceptable owing to the flexibility of the enzymes 4.

On the other hand, in the cases where the sizes of the small cavities of the mesoporous silica materials 3 were about 0.3 and 12.3 times the sizes of the enzymes 4 (namely, the cases where the average small cavity diameters were about 2.7 nm and 98.4 nm), the output response current declined as time passed, and the response became 40% or less after 66 days compared with that on the starting day. The cause is considered deactivation of the enzymes 4 by the change of the steric structures thereof or the like, the enzymes 4 having not been firmly fixed for the immobilization in the small cavities of the mesoporous silica material 3 by setting the sizes of the small cavities to fit the enzyme 4.

As a result, it was confirmed that the enzyme sensor 100 with excellent stability and repeatability could be made by setting the sizes of the small cavities of the mesoporous silica material 3 to be about 0.5 to 2.0 times (more preferably about 0.7 to 1.4 times) the sizes of the enzymes 4 to be immobilized.

From the results of the adsorption of the enzymes 4, the stability of the repeated measurement of the enzyme sensor 100, and the repeatability of the enzyme sensor 100, it was confirmed that the high-sensitive enzyme sensor 100 having excellent stability and repeatability could be made by setting the sizes of the small cavities of the mesoporous silica material 3 to be about 0.5 to 2.0 times (more preferably about 0.7 to 1.4 times, and most preferably almost equal to) the sizes of the enzymes 4 to be immobilized.

As described above, in the enzyme electrode 1 and the enzyme sensor 100 using the enzyme electrode 1 of the present invention, the enzyme electrode 1 includes the electrode 2, the mesoporous silica material 3 provided on the electrode 2, and the enzymes 4 immobilized in the small cavities of the mesoporous silica material 3, wherein the sizes of the small cavities of the mesoporous silica material 3 are set to be 0.5 to 2.0 times the sizes of the enzymes 4.

Accordingly, the enzymes 4 can be firmly fixed for the immobilization in the small cavities of the mesoporous silica material 3 with high degree of freedom. Therefore, the steric structures of the enzymes 4 are prevented from changing, and hence the enzyme electrode 1 with excellent stability and a longer operating life, and the enzyme sensor 100 using the enzyme electrode 1 can be provided.

Furthermore, the mesoporous silica material 3 is porous and has a very large specific surface area. Therefore, in the case where the mesoporous silica material 3 is used as the carrier, the enzymes 4 can be immobilized with more adsorption at a higher concentration compared with the case where the carrier having a smaller specific surface area than that of the mesoporous silica material 3 is used. In addition, the state of the enzymes 4 being properly dispersed can be maintained by firmly fixing the enzymes 4 for the immobilization in the small cavities of the mesoporous silica material 3, so that deactivation of the enzymes 4 caused by aggregation thereof and the like can be avoided. That is to say, by using, as the carrier, the mesoporous silica material 3 in which the sizes of the small cavities are set to be 0.5 to 2.0 times the sizes of the enzymes 4, the enzymes 4 can be immobilized with more adsorption at a higher concentration, and the deactivation of the enzymes 4 caused by the aggregation thereof and the like can be avoided, so that the enzyme electrode 1 with excellent stability and a longer operating life, and the enzyme sensor 100 using the enzyme electrode 1 can be provided.

Moreover, since the enzymes 4 are immobilized in the state of being properly dispersed, the transmission and diffusion of the substance is good and the enzyme reaction efficiently proceeds.

In the enzyme electrode 1 and the enzyme sensor 100 using the enzyme electrode 1 of the present invention, the mesoporous silica material 3 can be prevented from drying by introducing water molecules into the small cavities thereof, and has a role as a filter to enable sensing under the condition where the small cavities block molecules, impurities, biomolecules, and the like being larger than the diameters thereof. Accordingly, the enzymes 4 can be protected from causes of the deactivation (impurities and the like), so that resistance of the enzyme sensor 100 toward dryness, the impurities, and the like can improve.

In the enzyme electrode 1 and the enzyme sensor 100 using the enzyme electrode 1 of the present invention, the mesoporous silica material 3 has concentration action on the target substance of the enzymes 4, and hence, can detect a trace amount of the target substance in a gaseous phase or a liquid phase at very high sensitivity. Also, the transfer of the electron between the active centers of the enzymes 4 immobilized in the small cavities of the mesoporous silica material 3 and the electrode 2 is mediated and facilitated by using the electron carrier. As a result, a high-speed and high-sensitive response can be obtained. With these actions, the target substance can be detected at very high speed and high sensitivity by the electrochemical measurement, and the high-speed and high-sensitive enzyme sensor 100 having a longer operating life and the excellent stability can be made.

The enzyme sensor 100 using the enzyme electrode 1 of the present invention can be used as a sensor to detect a specific smell or gas by detecting a trace amount of the target substance at very high sensitivity in a gaseous phase or a liquid phase. Also, the enzyme sensor 100 using the enzyme electrode 1 of the present invention can be used as a sensor to detect a specific taste in a liquid phase, or as a sensor to detect production of a specific substance and/or the amount of the production thereof in a bioreactor.

The enzyme electrode 1 and the enzyme sensor 100 using the enzyme electrode 1 of the present invention can selectively measure the target substance at high speed and high sensitivity by setting a voltage of the working electrode (enzyme electrode 1) toward the reference electrode 400 to be a specific voltage, avoiding influence of an interfering substance. In particular, use of the electron carrier can decrease the set voltage and improve the selectivity.

In the enzyme electrode 1 and the enzyme sensor 100 using the enzyme electrode 1 of the present invention, the enzymes 4 which are an element for molecule discrimination need to be changed to another kind of enzymes in accordance with the target substance. For example, glucose oxidase or glucose dehydrogenase for glucose being the target substance (object for measurement), alcohol oxidase or alcohol dehydrogenase for ethanol being the object for measurement, formaldehyde oxidase or formaldehyde dehydrogenase for formaldehyde being the object for measurement, and a mixture of cholesterol esterase and cholesterol oxidase for the total cholesterol being the object for measurement can be used as the enzymes 4.

The present invention is not limited to the examples and can be appropriately modified without departing from the scope of the invention.

The potentiostat 500 is included in the enzyme sensor 100 . However, this is not limitation and the potentiostat 500 and the enzyme sensor 100 may be configured individually.

For the electrochemical measurement by the enzyme sensor 100, two electrodes (the working electrode and the counter electrode) formed on one substrate, or three electrodes (the working electrode, the counter electrode, and the reference electrode) formed on one substrate, may be used. Also, individually formed electrodes (the working electrode, the counter electrode, and the reference electrode) may be combined to use.

In the enzyme electrode 1, the enzyme protein complex C may be immobilized on the working electrode (the electrode 2), on a disc in the case of a disc electrode for example, over a plurality of the electrodes 2 (two electrodes or three electrodes, for example), around the electrode 2, or in a part of the electrolytic solution which is apart from the electrode 2.

In the examples, the enzyme sensor 100 detects the concentration of the target substance in a liquid phase. However, needless to say, the enzyme sensor 100 can also detect the concentration of the target substance in a gaseous phase.

Currently, as an eco-friendly future energy device, a biofuel cell is researched and developed. The cell uses biomass such as sugar and alcohol as an energy source, and extracts power generated by an electron which is produced at the time of decomposition of the energy source. The cell is expected as a next-generation clean energy conversion device. A sensor is for measuring an electric current by supplying a power source and a cell is for obtaining energy by extracting the electric current. Therefore, an art of a biosensor can be utilized for these as it is.

Main problems thereof at present are a low output current, low durability, and the like. However, because the increase of the enzyme-immobilized specific surface and the improvement of the stability of the enzymes can be achieved by using the enzyme electrode of the present invention as the electrode for the biofuel cell, the increase of the current density and the improvement of the stability of the electrode can be achieved, and therefore performance of the biofuel cell can dramatically improve.

### Brief Description of the Drawings

[FIG. 1] This is a perspective view for schematically showing the enzyme electrode of the present invention.
[FIG. 2] This shows the structure of formaldehyde dehydrogenase.

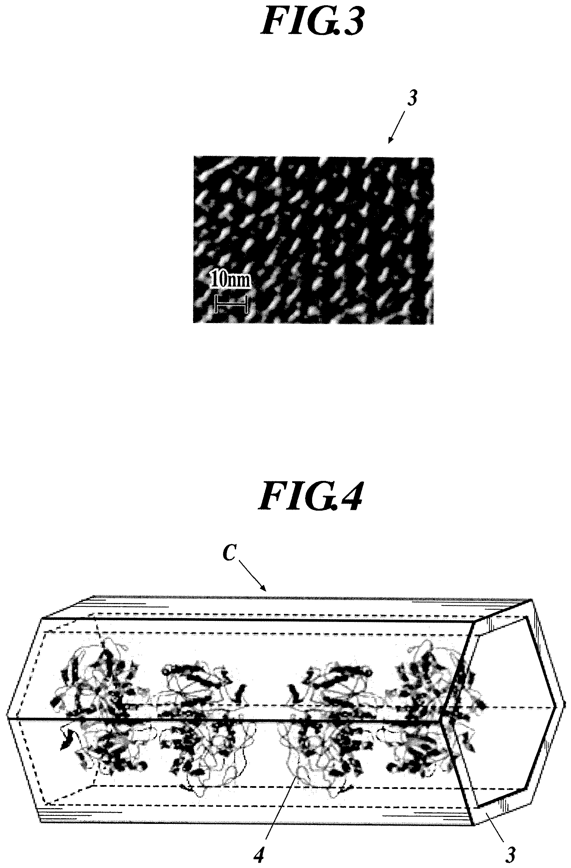

[FIG. 3] This is a TEM (transmission electron microscope) image of the mesoporous silica material.
[FIG. 4] This is a perspective view for schematically showing a main part of the enzyme protein complex formed by immobilizing the enzymes in the small cavities of the mesoporous silica material.
[FIG. 5] This is a perspective view for schematically showing an example of the enzyme protein complex formed by immobilizing the enzymes in the small cavities of the mesoporous silica material, and a main part of the enzyme protein complex.
[FIG. 6] This illustrates the principle of the electrochemical measurement for the concentration of the target substance in the sample at high speed and high sensitivity by the enzyme sensor using the enzyme electrode.
[FIG. 7] This shows the result (activity of the enzymes immobilized in the mesoporous silica material) obtained by the measurement using the enzyme sensor of the first example.
[FIG. 8] This illustrates the method for making the enzyme electrode.
[FIG. 9] This shows the enzyme sensor of the present invention schematically.
[FIG. 10] This shows the result (relationship between the concentration of the aqueous formaldehyde solution and the response current) obtained by the measurement using the enzyme sensor of the first example.
[FIG. 11] This shows the result (effectiveness of the introduction of the electron carrier into the small cavities of the mesoporous silica material) obtained by the measurement using the enzyme sensor of the first example.
[FIG. 12] This shows the result (response time of the enzyme sensor) obtained by the measurement using the enzyme sensor of the first example.
[FIG. 13] This shows the result (selectivity of the enzyme sensor) obtained by the measurement using the enzyme sensor of the first example.
[FIG. 14] This shows the result (repeatability of the enzyme sensor) obtained by the measurement using the enzyme sensor of the first example.
[FIG. 15] This shows the result (effectiveness of the immobilization of the enzymes in the mesoporous silica material) obtained by the measurement using the enzyme sensor of the first example.
[FIG. 16] This shows the result (activity of the enzymes immobilized in the mesoporous silica material) obtained by the measurement using the enzyme sensor of the second example.
[FIG. 17] This shows the result (relationship between the concentration of the aqueous formaldehyde solution and the response current) obtained by the measurement using the enzyme sensor of the second example.
[FIG. 18] This shows the result (effectiveness of the introduction of the electron carrier into the small cavities of the mesoporous silica material) obtained by the measurement using the enzyme sensor of the second example.
[FIG. 19] This shows the result (response time of the enzyme sensor) obtained by the measurement using the enzyme sensor of the second example.
[FIG. 20] This shows the result (selectivity of the enzyme sensor) obtained by the measurement using the enzyme sensor of the second example.
[FIG. 21] This shows the result (repeatability of the enzyme sensor) obtained by the measurement using the enzyme sensor of the second example.
[FIG. 22] This shows the result (effectiveness of the immobilization of the enzymes in the mesoporous silica material) obtained by the measurement using the enzyme sensor of the second example.
[FIG. 23] This shows the result (adsorption of the enzymes) obtained by the measurement using the mesoporous silica material of the third example.
[FIG. 24] This shows the result (repeated measurement stability of the enzyme sensor) obtained by the measurement using the enzyme sensor of the third example.
[FIG. 25] This shows the result (repeatability of the enzyme sensor) obtained by the measurement using the enzyme sensor of the third example.

### Explanations of Numerals

- 1: enzyme electrode
- 2: electrode
- 3: mesoporous silica material
- 4: enzymes
- 100: enzyme sensor

## Claims

1. An enzyme electrode comprising:
an electrode;
a mesoporous silica material provided on the electrode;
an enzyme immobilized in small cavities of the mesoporous silica material; and
an electron carrier to facilitate transfer of an electron between the enzyme and the electrode, the electron carrier being present in the small cavities of the mesoporous silica material,
wherein
a diameter of the small cavities is 0.5 to 2.0 times a diameter of the enzyme,
the mesoporous silica material is membranous and made of a porous anodized aluminum film having alumina small cavities each filled with a plurality of one-dimensional silica nanochannels which constitute a one-dimensional silica nanochannel assembly, and
the small cavities of the mesoporous silica material are cavities of the one-dimensional silica nanochannels.

2. The enzyme electrode according to claim 1, wherein a coenzyme to catalyze expression of activity of the enzyme is present in the small cavities of the mesoporous silica material.

3. The enzyme electrode according to claim 1 or 2 wherein a water molecule necessary for the expression of the activity of the enzyme is present in the small cavities of the mesoporous silica material.

4. The enzyme electrode according to any one of claims 1 to 3, further comprising a film to transmit a specific substance selectively .

5. The enzyme electrode according to any one of claims 1 to 4, further comprising a film not to transmit a specific substance selectively.

6. The enzyme electrode according to any one of claims 1 to 5, wherein the enzyme is composed of two kinds of enzymes.

7. An enzyme sensor, comprising the enzyme electrode according to any one of claims 1 to 6, wherein a target substance is detected by an electrochemical measurement using the enzyme electrode.

## Patentansprüche

1. Enzymelektrode, umfassend:
eine Elektrode;
ein mesoporöses Siliciumdioxidmaterial, das auf der Elektrode bereitgestellt ist;
ein Enzym, das in kleinen Hohlräumen des mesoporösen Siliciumdioxidmaterials immobilisiert ist; und
ein Elektronenüberträger, um den Transfer eines Elektrons zwischen dem Enzym und der Elektrode zu ermöglichen,
wobei der Elektronenträger in den kleinen Hohlräumen des mesoporösen Siliciumdioxidmaterials vorhanden ist,
wobei ein Durchmesser der kleinen Hohlräume 0,5 bis 2 Mal der Durchmesser des Enzyms ist,
das mesoporöse Siliciumdioxidmaterial membranartig ist und aus einem porösen anodisiertem Aluminiumfilm mit kleinen Aluminiumoxidhohlräumen besteht, jeweils gefüllt mit einer Vielzahl von eindimensionalen Siliciumdioxid-Nanokanälen, die eine eindimensionale Siliciumdioxid-Nanokanal-Anordnung bilden, und
die kleinen Hohlräume des mesoporösen Siliciumdioxidmaterials Hohlräume des eindimensionalen Siliciumdioxid-Nanokanals sind.

2. Enzymelektrode nach Anspruch 1, wobei ein Coenzym in den kleinen Hohlräumen des mesoporösen Siliciumdioxidmaterials vorhanden ist, um die Expression der Aktivität des Enzyms zu katalysieren.

3. Enzymelektrode nach Anspruch 1 oder 2, wobei ein Wassermolekül, notwendig für die Expression der Aktivität des Enzyms, in den kleinen Hohlräumen des mesoporösen Siliciumdioxidmaterials vorhanden ist.

4. Enzymelektrode nach einem der Ansprüche 1 bis 3, weiter umfassend einen Film, um eine spezifische Substanz selektiv zu übertragen.

5. Enzymelektrode nach einem der Ansprüche 1 bis 4, weiter umfassend einen Film, um eine spezifische Substanz selektiv nicht zu übertragen.

6. Enzymelektrode nach einem der Ansprüche 1 bis 5, wobei das Enzym aus zwei Arten von Enzymen zusammengesetzt ist.

7. Enzymsensor, umfassend die Enzymelektrode nach einem der Ansprüche 1 bis 6, wobei eine Zielsubstanz durch eine elektrochemische Messung unter Verwenden der Enzymelektrode detektiert wird.

## Revendications

1. Électrode enzymatique comprenant :
une électrode ;
un matériau de silice mésoporeuse situé sur l'électrode;
une enzyme immobilisée dans de petites cavités du matériau de silice mésoporeuse; et
un transporteur d'électron pour faciliter le transfert d'un électron entre l'enzyme et l'électrode,
le transporteur d'électron étant présent dans les petites cavités du matériau de silice mésoporeuse,
dans laquelle un diamètre des petites cavités est égal à 0,5 fois à 2 fois un diamètre de l'enzyme,
le matériau de silice mésoporeuse est membraneux et formé d'un film d'aluminium anodisé poreux comportant de petites cavités d'oxyde d'aluminium remplies chacune d'une pluralité de nanocanaux de silice unidimensionnels qui constituent un ensemble de nanocanaux de silice unidimensionnels, et
les petites cavités du matériau de silice mésoporeuse sont des cavités des nanocanaux de silice unidimensionnels.

2. Électrode enzymatique selon la revendication 1, dans laquelle une coenzyme destinée à catalyser l'expression de l'activité de l'enzyme est présente dans les petites cavités du matériau de silice mésoporeuse.

3. Électrode enzymatique selon la revendication 1 ou 2, dans laquelle une molécule d'eau nécessaire pour l'expression de l'activité de l'enzyme est présente dans les petites cavités du matériau de silice mésoporeuse.

4. Électrode enzymatique selon l'une quelconque des revendications 1 à 3, comprenant, en outre, un film destiné à transmettre une substance spécifique de manière sélective.

5. Électrode enzymatique selon l'une quelconque des revendications 1 à 4, comprenant, en outre, un film non destiné à transmettre une substance spécifique de manière sélective.

6. Électrode enzymatique selon l'une quelconque des revendications 1 à 5, dans laquelle l'enzyme est composée de deux types d'enzymes.

7. Capteur enzymatique comprenant l'électrode enzymatique selon l'une quelconque des revendications 1 à 6, dans lequel une substance cible est détectée par une mesure électrochimique en utilisant l'électrode enzymatique.
